# EUROPÄISCHE PATENTSCHRIFT

(11) **EP 3 806 914 B1**
(45) Veröffentlichungstag und Bekanntmachung des Hinweises auf die Patenterteilung: **11.03.2026**
(21) Anmeldenummer: 19729259.2
(22) Anmeldetag: 05.06.2019
(51) Int. Cl.: A61L 9/14, B05B 3/10, B05B 12/08

(54) **VORRICHTUNG ZUM DESINFIZIEREN ZUMINDEST EINES RAUMS, INSBESONDERE PERSONEN-AUFENTHALTSRAUMS, MIT EINEM ZERSTÄUBER**
DEVICE FOR DISINFECTING AT LEAST ONE ROOM, IN PARTICULAR A PEOPLE'S LOUNGE, BY MEANS OF AN ATOMIZER
DISPOSITIF DE DÉSINFECTION D'AU MOINS UNE CHAMBRE, EN PARTICULIER D'UNE CHAMBRE DE SÉJOUR DE PERSONNES, POURVU D'UN PULVÉRISATEUR

(30) Priorität: 13.06.2018 DE 102018114179
(43) Veröffentlichungstag der Anmeldung: 21.04.2021
(73) Patentinhaber: Dürr Systems AG, 74321 Bietigheim-Bissingen (DE)
(72) Erfinder: HÄUSSERMANN, Patrick, 71404 Korb (DE); SEIZ, Bernhard, 74348 Lauffen (DE); PREUSS, Kevin, 75417 Mühlacker (DE); KRUMMA, Harry, 74357 Bönnigheim (DE); BAUMANN, Michael, 74223 Flein (DE); DION, Mark, E., Mi 48080 (US)
(74) Vertreter: v. Bezold & Partner Patentanwälte - PartG mbB
(86) Internationale Anmeldenummer: PCT/EP2019/064683
(87) Internationale Veröffentlichungsnummer: WO 2019/238503

(56) Entgegenhaltungen:
- EP-A1- 2 441 523
- WO-A1-2004/030828
- FR-A- 1 310 867
- FR-A1- 2 886 559
- JP-B2- 2 852 823
- US-A- 3 192 167

## Beschreibung

Die Erfindung betrifft eine Vorrichtung zum Desinfizieren zumindest eines Raums, vorzugsweise für eine oder mehrere Personen, z. B. einen Aufenthaltsraum, Lagerraum, Kühlraum und/oder Behandlungsraum, zum Beispiel einen Behandlungsraum eines Gebäudes, insbesondere ein Krankenzimmer, ein Patientenzimmer und/oder ein Operationssaal, mit einem Zerstäuber zweckmäßig zum Zerstäuben eines Desinfektionsmittels in den Raum. Der Raum ist vorzugsweise durch eine oder mehrere Personen begehbar.

Es ist üblich, dass z. B. Krankenzimmer oder Operationssäle in Krankenhäusern oder Arztpraxen regelmäßig desinfiziert werden, um insbesondere Bakterien, Viren, Pilze, etc. zu beseitigen. US 2014/0119992 A1 beschreibt hierzu ein Verfahren, wobei eine Scheibe, eine Düse oder ein piezoelektrischer Ultraschall-Zerstäuber einen Nebel aus Desinfektionsmittel erzeugt.

Aus FR 1 310 867 A, US 3,192,167 A, EP 2 441 523 A1, FR 2 886 559 A1, JP 2 852 823 B2 und WO 2004/030828 A1 sind auch Vorrichtungen mit Rotationszerstäubern bekannt, die zum Zerstäuben von Desinfektionsmitteln verwendbar sind.

Eine Aufgabe der Erfindung ist es, eine Vorrichtung zum Desinfizieren zumindest eines vorzugsweise durch eine oder mehrere Personen begehbaren Raums mit verbesserter Leistung und/oder verbesserten Zerstäubungseigenschafften zu schaffen.

Diese Aufgabe kann mit den Merkmalen des unabhängigen Anspruchs gelöst werden. Vorteilhafte Weiterbildungen der Erfindung können den Unteransprüchen und der folgenden Beschreibung bevorzugter Ausführungsformen der Erfindung entnommen werden.

Die Erfindung betrifft eine Vorrichtung zum Desinfizieren zumindest eines Raums, z. B. für eine oder mehrere Personen, vorzugsweise eines Aufenthaltsraums, Lagerraums, Kühlraums und/oder Behandlungsraums (zweckmäßig für Mensch oder Tier), zum Beispiel eines Behandlungsraums eines Gebäudes, insbesondere eines Krankenzimmers, eines Patientenzimmers und/oder eines Operationssaals, z. B. eines Krankenhauses oder einer Arztpraxis. Der Aufenthaltsraum kann allerdings auch z. B. Schulräume, Bade- oder WC-Räume, sanitäre Anlagen oder Abteile von Flugzeugen, Schiffen oder anderen Fahrzeugen umfassen. Der Aufenthaltsraum kann vorzugsweise auch einen veterinären Aufenthaltsraum und/oder einen Warteraum umfassen.

Die Vorrichtung umfasst einen zweckmäßigen Zerstäuber, mit einem rotierbaren Glockenteller (insbesondere eine sogenannte Hochrotationsglocke) zum Zerstäuben eines Desinfektionsmittels in den Raum. Der Glockenteller ist vorzugsweise im Wesentlichen trichterförmig ausgebildet, kann aber auch z. B. im Wesentlichen scheibenförmig oder auf andere Weise ausgeführt sein.

Die Erfindung beruht unter anderem auf der Erkenntnis, einen rotierbaren Glockenteller zum Zerstäuben des Desinfektionsmittels in den Raum zu verwenden. Glockenteller und deren Wirkweise sind aus dem technischen Bereich der Lackierung von Kraftfahrzeugkarosserien mittels Rotationszerstäubern bekannt. Der Glockenteller dient dabei dazu, den Lack Zentrifugalkraft-basiert zu zerstäuben und in Form eines Lack-Sprühstrahls zur Lack-Bahnerzeugung auf die Kraftfahrzeugkarosserien abzugeben. Überraschend wurde erkannt, dass Glockenteller sich auch zur Zerstäubung von Desinfektionsmittel eignen und dabei insbesondere eine sehr gute Zerstäubungsleistung und/oder sehr gute Zerstäubungseigenschafften aufweisen.

Es ist möglich, dass die Vorrichtung einen Elektromotor und/oder eine z. B. pneumatisch angetriebe Turbine, insbesondere Luftturbine, zum Antreiben des Glockentellers aufweist, vorzugsweise über eine hohle Antriebswelle.

Die Vorrichtung kann z. B. zumindest ein Gebläse (z. B. Ventilator) aufweisen, wobei das Gebläse zum Transportieren und Verteilen von mittels des Glockentellers zerstäubtem Desinfektionsmittel dient. Ein Gebläse kann z. B. angeordnet sein, um das Desinfektionsmittel im Wesentlichen nach oben zu verteilen. Ein Gebläse kann z. B. angeordnet sein, um das Desinfektionsmittel nach seitlich außen zu verteilen. Eines dieser Gebläse kann zusätzlich genutzt werden, um den Elektromotor und/oder die Luftturbine zu kühlen.

Es ist möglich, dass der Glockenteller, der Elektromotor, die Luftturbine und/oder ein Gebläse zumindest abschnittsweise in einem Zerstäubergehäuse untergebracht ist.

Die Vorrichtung weist eine Desinfektionsmittel-Leitung auf, die mit dem Glockenteller in Verbindung bringbar ist, um dem Glockenteller das Desinfektionsmittel zuzuführen.

Es ist vorgesehen, dass die Vorrichtung eine zweckmäßig lösbare oder unlösbare Verbindungseinrichtung zum Verbinden mit einem Behälter zur Aufnahme des Desinfektionsmittels aufweist und die Verbindungseinrichtung über die Desinfektionsmittel-Leitung mit dem Glockenteller in Verbindung bringbar ist, um dem Glockenteller das Desinfektionsmittel zuzuführen.

Die Vorrichtung kann vorzugsweise zumindest eine zweckmäßig elektrisch, hydraulisch oder pneumatisch betriebene Fördereinrichtung zum Fördern und/oder Dosieren des Desinfektionsmittels für den Desinfektionsprozess aufweisen.

Es ist möglich, dass die Vorrichtung zumindest eine Durchflussmesseinrichtung (z. B. einen oder mehrere Sensoren) zum vorzugsweise kontaktlosen Messen eines Volumenstroms vorzugsweise des Desinfektionsmittels und/oder eines Spülmittels aufweist.

Eine Durchflussmesseinrichtung kann z. B. zwischen dem Glockenteller und einem stromabwärts der Fördereinrichtung angeordneten Glockenteller-Ventil (Zerstäuberventil) angeordnet sein, wobei alternativ oder ergänzend z. B. eine Durchflussmesseinrichtung zwischen der Fördereinrichtung und einem stromabwärts der Fördereinrichtung angeordneten Glockenteller-Ventil angeordnet sein kann.

Die Vorrichtung weist einen Aufnahmebehälter zur Aufnahme und vorzugsweise Rückgewinnung von durch den Desinfektionsprozess entstehendem Verlustmittel (z. B. nicht ausgegebenes Desinfektionsmittel, Spülmittel, abgeschiedene Feuchtigkeit, etc.) auf.

Der Aufnahmebehälter kann vorzugsweise zur Aufnahme von an oder in dem Zerstäubergehäuse aus dem Desinfektionsmittel abgeschiedener Flüssigkeit dienen, wobei z. B. die abgeschiedene Flüssigkeit insbesondere über einen Trichter dem Aufnahmebehälter zugeführt werden kann.

Die Vorrichtung kann z. B. zumindest einen Luftentfeuchter zur Entfeuchtung von Luft in dem Raum aufweisen, z. B. mit einer Gesamt-Entfeuchtungsleistung von z. B. über 600 oder über 700 Kubikmeter pro Stunde.

Der zumindest eine Luftentfeuchter kann vorzugsweise mit dem Aufnahmebehälter in Verbindung gebracht werden, um aus der Luft entzogene Feuchtigkeit (Flüssigkeit) dem Aufnahmebehälter zuzuführen.

Es ist möglich, dass die Fördereinrichtung über eine Leitung mit dem Aufnahmebehälter in Verbindung bringbar ist, um über die Leitung Flüssigkeit, z. B. Spülmittel und/oder Desinfektionsmittel, dem Aufnahmebehälter zuzuführen, insbesondere dann, wenn an einem Überdruckventil Überdruck anliegt. Das Überdruckventil kann z. B. in der Leitung zwischen der Fördereinrichtung und dem Aufnahmebehälter angeordnet sein oder in der Desinfektionsmittel-Leitung stromabwärts der Fördereinrichtung oder an oder in der Fördereinrichtung. Die Leitung kann z. B. direkt an die Fördereinrichtung angeschlossen sein.

Es ist vorgesehen, dass die Vorrichtung eine Verlustmittel-Leitung aufweist und z. B. der Aufnahmebehälter über die Verlustmittel-Leitung entleerbar ist und/oder mit der Verbindungseinrichtung in Verbindung bringbar ist.

Es ist möglich, dass Verlustmittel aus dem Aufnahmebehälter über die Verbindungseinrichtung dem zuvor im Desinfektionsprozess entleerten Desinfektionsmittel-Behälter zur Entsorgung zuführbar ist.

Das Verlustmittel kann auf seinem Weg vom Aufnahmebehälter zur Verbindungseinrichtung vorzugsweise zumindest eines von Folgendem passieren: eine Filtereinrichtung zur Filterung des Verlustmittels, die Fördereinrichtung, ein Aufnahmebehälter-Ventil, das vorzugsweise stromabwärts der Filtereinrichtung und/oder stromaufwärts der Fördereinrichtung angeordnet ist, ein Verlustmittel-Rückström-Ventil, das vorzugsweise stromabwärts der Fördereinrichtung und/oder stromaufwärts der Verbindungseinrichtung angeordnet ist, und/oder eine Durchflussmesseinrichtung zum Messen eines Volumenstroms, vorzugsweise des Desinfektionsmittels und/oder eines Spülmittels.

Die Vorrichtung kann z. B. einen zweckmäßig lösbaren oder unlösbaren Spülmittelanschluss zur Verbindung mit einem Spülmittel-Behälter zur Aufnahme eines Spülmittels aufweisen, um in einem Spülmodus zumindest abschnittsweise die Desinfektionsmittel-Leitung und/oder die Verlustmittel-Leitung mit dem Spülmittel zu spülen.

Der Spülmittelanschluss kann z. B. über eine Spülmittel-Leitung mit der Desinfektionsmittel-Leitung in Verbindung bringbar sein, wobei vorzugsweise die Spülmittel-Leitung vor Mündung in die Desinfektionsmittel-Leitung ein Spülmittel-Ventil umfassen kann.

Es ist möglich, dass die Vorrichtung für den Desinfektionsprozess in einem Desinfizierungs-Modus zum Desinfizieren des Raums betreibbar ist.

Es ist möglich, dass die Vorrichtung in einem Rückgewinnungs-Modus zum Rückgewinnen von durch den Desinfektionsprozess entstehendem Verlustmittel betreibbar ist und/oder in einem Spülmodus zum zumindest abschnittsweisen Spülen der Desinfektionsmittel-Leitung und/oder der Verlustmittel-Leitung betreibbar ist.

Die Fördereinrichtung kann z. B. im Rückgewinnungs-Modus zum Fördern des Verlustmittels aus dem Aufnahmebehälter dienen und/oder im Spülmodus zum Fördern des Spülmittels aus dem Spülmittel-Behälter dienen und im Desinfektionsprozess zum Zuführen des Desinfektionsmittels zum Glockenteller dienen.

Es ist möglich, dass die Verlustmittel-Leitung an zwei Knotenpunkten mit der Desinfektionsmittel-Leitung verbunden ist.

Ein Knotenpunkt ist vorzugsweise zwischen der Fördereinrichtung und einem in der Desinfektionsmittel-Leitung zweckmäßig stromabwärts der Fördereinrichtung angeordneten Glockenteller-Ventil angeordnet. Alternativ oder ergänzend ist ein Knotenpunkt zwischen der Fördereinrichtung und einem in der Desinfektionsmittel-Leitung zweckmäßig stromaufwärts der Fördereinrichtung angeordneten Desinfektionsmittel-Ventil angeordnet ist.

Die Spülmittel-Leitung kann an einem Knotenpunkt mit der Desinfektionsmittel-Leitung verbunden sein. Der Knotenpunkt ist vorzugsweise zwischen der Förderreinrichtung und einem in der Desinfektionsmittel-Leitung stromaufwärts der Fördereinrichtung angeordneten Desinfektionsmittel-Ventil angeordnet.

Es ist möglich, dass die Vorrichtung einen Multi-Behälter zur Aufnahme des Desinfektionsmittels für den Desinfektionsprozess aufweist und z. B. der Multi-Behälter zusätzlich zur Aufnahme von Spülmittel für den Spülmodus dient, so dass vorzugsweise der Multi-Behälter für den Desinfektionsprozess mit dem Desinfektionsmittel und/oder für den Spülmodus mit dem Spülmittel zweckmäßig nacheinander befüllbar und betreibbar ist.

Der Multi-Behälter kann z. B. in die Desinfektionsmittel-Leitung und/oder die Spülmittel-Leitung integrierbar sein.

Es ist möglich, dass der Multi-Behälter, vorzugsweise über das Desinfektionsmittel-Ventil, mit der Verbindungseinrichtung in Verbindung bringbar ist und/oder, vorzugsweise über das Spülmittel-Ventil, mit dem Spülmittelanschluss in Verbindung bringbar ist.

Es ist möglich, dass der Glockenteller mit einer hohlen Antriebswelle zum Antreiben des Glockentellers verbunden ist. Die hohle Antriebswelle wird vorzugsweise durch den Elektromotor und/oder die Luftturbine angetrieben.

Eine Zuleitung in der hohlen Antriebswelle ist vorzugsweise Teil der Desinfektionsmittel-Leitung.

Die Zuführung des Desinfektionsmittels zum Glockenteller kann vorzugsweise durch die hohle Antriebswelle hindurch erfolgen.

Die Zuleitung in der hohlen Antriebswelle kann insbesondere eine z. B. anmontierte Desinfektionsmittel-Austrittsdüse umfassen, wobei die Desinfektionsmittel-Austrittsdüse vorzugsweise in einer Basis des Glockentellers aufgenommen ist und/oder einen reduzierten Durchlassquerschnitt für das Desinfektionsmittel im Vergleich zum Durchlassquerschnitt der hohlen Antriebswelle aufweisen kann.

Der Glockenteller kann mit einer zweckmäßig mit dem Glockenteller mitrotierbaren Platte zusammenwirken, wobei das Desinfektionsmittel mittels der Platte zum Glockenteller verteilt wird.

Die Platte ist vorzugsweise am Glockenteller montiert.

Der Glockenteller ist vorzugsweise an der hohlen Antriebswelle montiert.

Der Glockenteller und die Platte funktionieren vorzugsweise im Wesentlichen wie folgt: Das aus der Zuleitung in der hohlen Antriebswelle, insbesondere der Desinfektionsmittel-Austrittsdüse, austretende Desinfektionsmittel trifft auf die rotierende Platte und wird dadurch Zentrifugalkraft-basiert auf eine Innenfläche des rotierenden Glockentellers verteilt, von wo aus das Desinfektionsmittel zu einer Glockentellerabspritzkante zum Abspritzen und Zerstäuben geführt wird.

Die Platte ist vorzugsweise eine zweckmäßig im Wesentlichen scheibenförmige Prall- und/oder Verteilerplatte.

Die Platte ist vorzugsweise koaxial zum Glockenteller und/oder der hohlen Antriebswelle angeordnet.

Die Platte ist vorzugsweise drehfest mit dem Glockenteller verbunden.

Die Platte kann z. B. in einem axialen Abstand von zwischen ungefähr 0,05mm bis ungefähr 5mm, typischerweise zwischen 0,75 mm und 2 mm, von einer Austrittsmündung der hohlen Antriebswelle, insbesondere der Desinfektionsmittel-Austrittsdüse, angeordnet sein.

Es ist möglich, dass das Desinfektionsmittel auf seinem Weg zum Glockenteller zumindest eines von Folgendem passiert: ein Desinfektionsmittel-Ventil zweckmäßig stromaufwärts der Fördereinrichtung, ein Glockenteller-Ventil zweckmäßig stromabwärts der Fördereinrichtung, die Fördereinrichtung, den Elektromotor oder die Luftturbine, die hohle Antriebswelle (insbesondere deren Zuleitung) z. B. mit Desinfektionsmittel-Austrittsdüse, die Platte, und/oder zumindest eine Durchflussmesseinrichtung zum Messen eines Volumenstroms, insbesondere eines Desinfektionsmittel- und/oder Spülmittel-Volumenstroms.

Die Fördereinrichtung kann z. B. zumindest eine Pumpe und/oder zumindest ein Kolbensystem umfassen, zweckmäßig elektrisch, pneumatisch oder hydraulisch angetrieben.

Es ist möglich, dass das Spülmittel im Spülmodus zumindest eines von Folgendem passiert: das Spülmittel-Ventil, die Fördereinrichtung, einen Teilabschnitt der Desinfektionsmittel-Leitung, die hohle Antriebswelle, einen Teilabschnitt der Verlustmittel-Leitung, zumindest eine Durchflussmesseinrichtung, das in der Desinfektionsmittel-Leitung zweckmäßig stromabwärts der Fördereinrichtung angeordnete Glockenteller-Ventil, den Elektromotor oder die Luftturbine, den Glockenteller, die Platte, das in der Verlustmittel-Leitung zweckmäßig stromabwärts der Fördereinrichtung angeordnete Verlustmittel-Rückström-Ventil, und/oder die die Verbindungseinrichtung.

Es ist möglich, dass die Fördereinrichtung und insbesondere die Pumpe zur Förderung von zumindest einem von Folgendem dient: des Desinfektionsmittels aus dem Desinfektionsmittel-Behälter, des Spülmittels aus dem Spülmittel-Behälter, und/oder des Verlustmittels aus dem Aufnahmebehälter.

Das Kolbensystem kann z. B. zumindest eines von Folgendem umfassen: ein Desinfektionsmittel-Kolbensystem, wobei vorzugsweise der Desinfektionsmittel-Behälter in das Desinfektionsmittel-Kolbensystem integrierbar ist, ein Spülmittel-Kolbensystem, wobei vorzugsweise der Spülmittel-Behälter in das Spülmittel-Kolbensystem integrierbar ist, und/oder ein Desinfektionsmittel-Spülmittel-Kolbensystem, wobei vorzugsweise der Multi-Behälter in das Desinfektionsmittel-Spülmittel-Kolbensystem integrierbar ist.

Es ist möglich, dass die Vorrichtung zumindest ein zweckmäßig stromaufwärts des Glockentellers angeordnetes Gebläse zum Transportieren und Verteilen von mittels des Glockentellers zerstäubtem Desinfektionsmittel aufweist und/oder zumindest ein zweckmäßig stromabwärts des Glockentellers angeordnetes Gebläse zum Transportieren und Verteilen von mittels des Glockentellers zerstäubtem Desinfektionsmittel aufweist.

Das Gebläse zweckmäßig stromaufwärts des Glockentellers kann z. B. koaxial zum Glockenteller angeordnet sein, um z. B. das Desinfektionsmittel aufwärts zu einer Decke des Raums zu verteilen und/oder um den Elektromotor oder die Luftturbine zu kühlen.

Das Gebläse zweckmäßig stromabwärts des Glockentellers ist vorzugsweise nicht-koaxial zum Glockenteller und/oder seitlich neben dem Glockenteller angeordnet, um z. B. das Desinfektionsmittel zur Seite hin, z. B. zu einer oder mehreren Wänden des Raums, zu verteilen.

Es ist möglich, dass die Vorrichtung eine Feuchtigkeitsmesseinrichtung (z. B. einen oder mehrere Sensoren) z. B. mit Regelwirkung und/oder Kontrollwirkung auf einen Desinfizierungsprozess der Vorrichtung aufweist, wobei die Feuchtigkeitsmesseinrichtung insbesondere zum Messen einer Feuchtigkeit in dem Raum dient. Die Regelwirkung kann z. B. zweckmäßig mit Rückkopplung oder ohne Rückkopplung ausgeführt sein.

Es ist möglich, dass die Vorrichtung eine Temperaturmesseinrichtung (z. B. einen oder mehrere Sensoren) z. B. mit Regelwirkung und/oder Kontrollwirkung auf einen Desinfizierungsprozess der Vorrichtung aufweist, wobei die Temperaturmesseinrichtung insbesondere zum Messen einer Temperatur in dem zumindest einen Raum dient. Die Regelwirkung kann z. B. zweckmäßig mit Rückkopplung oder ohne Rückkopplung ausgeführt sein.

Die Vorrichtung kann z. B. zumindest eine Füllstandsmesseinrichtung (z. B. einen oder mehrere Sensoren) zum Messen eines in dem Desinfektionsmittel-Behälter vorliegenden Füllstands, zum Messen eines in dem Aufnahmebehälter vorliegenden Füllstands und/oder zum Messen eines in dem Spülmittel-Behälter vorliegenden Füllstands aufweisen. Die Füllstandsmesseinrichtung kann den Füllstand z. B. über das Gewicht des jeweiligen Behälters und somit vorzugsweise Schwerkraft-basiert messen oder aber z. B. über Füllstandsmesssensoren. Nur bei i. O. Füllstand kann z. B. Prozessfreigabe erfolgen.

Es ist möglich, dass der Desinfektionsmittel-Behälter, der Spülmittel-Behälter und/oder der Multi-Behälter als wechselbarer Einweg-Behälter ausgeführt ist oder als fest in die Vorrichtung integrierter z. B. nachfüllbarer Behälter zweckmäßig in Form eines nachfüllbaren Tanks ausgeführt ist.

Es ist möglich, dass der Glockenteller im Betrieb eine Drehzahl von zwischen mindestens 30.000 bis z. B. maximal 70.000 Umdrehungen pro Minute aufweist.

Der Glockenteller und folglich die Vorrichtung kann z. B. eine Zerstäubungsleistung von mindestens 50, 100, 150 oder 200 Milliliter pro Minute aufweisen und/oder ein umgewälztes Luftvolumen von zumindest 0,3, 1,5, 10 oder 15 Kubikmeter pro Minute aufweisen.

Der Glockenteller weist vorzugsweise eine im Wesentlichen ringförmige Glockentellerabspritzkante zum Abspritzen des Desinfektionsmittels auf, wobei das Desinfektionsmittel zweckmäßig an der Glockentellerabspritzkante zerstäubt wird. Die Glockentellerabspritzkante kann z. B. einen Durchmesser zwischen 40mm und 120mm aufweisen.

Es ist möglich, dass die Vorrichtung zumindest eine Raumvermessungseinrichtung (z. B. einen oder mehrere Sensoren) zum Vermessen des Raums aufweist, wobei ein Desinfizierungsprozess der Vorrichtung in Abhängigkeit von Messdaten der Raumvermessungseinrichtung erfolgt oder eine Einleitung eines Desinfizierungsprozesses der Vorrichtung unterbunden wird, z. B. wenn erkannt wird, dass die Vorrichtung unsachgemäß im Raum positioniert ist oder der Raum für einen Desinfizierungsprozesses ungeeignet erscheint.

Die Vorrichtung kann z. B. einen Gasleitring, zweckmäßig mit einer oder mehreren Leckageöffnungen aufweisen. Der Gasleitring kann vorzugsweise die Leitung eines Gases (insbesondere Luft) an der Außenfläche (z. B. des Glockentellers) zur Aufgabe haben. Das Gas kann dabei z. B. auf das vom Glockenteller abgegebene Desinfektionsmittel treffen, insbesondere um das Desinfektionsmittel zu formen und/oder dessen Tröpfchengröße zu reduzieren.

Der Gasleitring ist vorzugsweise so angeordnet, dass das Gas über eine Außenfläche des Glockentellers auf das mittels der Glockentellerabspritzkante abgegebene Desinfektionsmittel trifft.

Der Gasleitring kann eine Vielzahl an ringförmig angeordneten Leckageöfnungen aufweisen.

Der Gasleitring kann z. B. eine Basis des Glockentellers (z. B. einen Sockelabschnitt) und/oder einen Teil einer Außenfläche des Glockentellers zweckmäßig im Wesentlichen ringförmig ummanteln.

Es ist möglich, dass die Vorrichtung zumindest eine Erkennungseinrichtung (z. B. einen oder mehrere Sensoren, Erkennung mittels RFID ("radio-frequency identification") oder Bar-Code, etc.) umfasst, zum Erkennen des Desinfektionsmittels (z. B. Art, Zusammensetzung, etc.), zum Erkennen des Spülmittels (z. B. Art, Zusammensetzung, etc.) und/oder zum Erkennen einer Drehzahl des Glockentellers (z. B. einer zu langsamen Drehzahl oder sogar einem nicht-rotierbaren Glockenteller oder einer zu schnellen Drehzahl). Im Kontext der Erfindung kann das Erkennen der Drehzahl des Glockentellers auch z. B. ein Erkennen der Drehzahl der hohlen Antriebswelle, des Elektromotors oder der Luftturbine oder das Herleiten aus anderen geeigneten Kennwerten umfassen. Das Erkennen kann somit im Kontext der Erfindung zweckmäßig unmittelbar oder mittelbar erfolgen.

Es ist möglich, dass Vorrichtung ihren Betrieb unterbricht oder unterbindet (z. B. nicht startet), wenn die Erkennungseinrichtung ein unpassendes Desinfektionsmittel, ein unpassendes Spülmittel und/oder eine unpassende Drehzahl erkennt. So kann z. B. eine Zufuhr an Desinfektionsmittel unterbrochen oder unterbunden werden, insbesondere wenn der Glockenteller nicht oder nicht mit einer vordefinierten Drehzahl rotiert und/oder ein falsches Desinfektionsmittel erkannt wird.

Es ist möglich, dass das zerstäubte Desinfektionsmittel Tröpfchen mit einer Größer, insbesondere einem Durchmesser, von zwischen im Wesentlichen 1µm und 50 µm, vorzugsweise zwischen 3 µm und 10 µm aufweist.

Es kann z. B. folgende Tropfenverteilung (Bereich volumetrisch/numerisch) erzielt werden:
Numerische Verteilung DN (50) = vorzugsweise 0,1 µm bis 10 µm
Volumetrische Verteilung DV (50) = vorzugsweise 5 µm bis 50 µm

Tropfenverteilung zweckmäßig gemessen mit Messsystem *Malvern Instruments* über 1000 Messpunkte je Sekunde in einem Abstand von 300mm über eine Strecke von 100mm zum Zerstäuber, insbesondere Glockenteller.

Die Vorrichtung kann eine z. B. portable Steuereinheit (z. B. Tablet, App, etc.) aufweisen, mittels der die Vorrichtung über eine Kabelverbindung oder kabellos (z.B. WLAN, Bluetooth, etc.) steuerbar ist.

Zu erwähnen ist, dass die Vorrichtung üblicherweise durch eine Bedienperson in einem Raum platziert wird, so dass die Vorrichtung dort den Desinfizierungsprozess ausführen kann. Während des Desinfizierungsprozess sollte sich zweckmäßig aber keine Person im Raum befinden.

Zu erwähnen ist außerdem, dass die hierin erwähnten Behälter, insbesondere der Desinfektionsmittel-Behälter und/oder der Spülmittel-Behälter, als Einweg-Behälter ausgeführt sein können oder aber als nachfüllbarer Tank zur Mehrfachnutzung ausgeführt sein können. Folglich kann vorzugsweise die Verbindungseinrichtung und/oder der Spülmittelanschluss zweckmäßig eine unlösbare Verbindung z. B. zu einem Tank darstellen oder aber eine lösbare Verbindung z B. zu einem Einweg-Behälter. Bei der unlösbaren Verbindung kann es sich z. B. um eine einstückig-integrale Verbindung, eine Klebeverbindung oder eine Schweißverbindung, etc. zwischen Behälter/Tank und Leitung, insbesondere Desinfektionsmittel-Leitung oder Spülmittel-Leitung handeln.

Zu erwähnen ist des Weiteren, dass die Vorrichtung vorzugsweise einen Glockenteller umfasst. Die Erfindung umfasst allerdings auch Ausführungsformen mit einer anderen Zerstäubervariante, so dass der Glockenteller optional ist und durch ein anderes Zerstäuberorgan ersetzt werden kann.

Das Spülmittel-Ventil, das Aufnahmebehälter-Ventil, das Verlustmittel-Rückführ-Ventil und/oder das Glockenteller-Ventil ist vorzugsweise steuerbar, z. B. elektrisch, pneumatisch, magnetisch oder hydraulisch.

Die ringförmige Glockentellerabspritzkante kann z. B. einen Durchmesser von zwischen 65 mm bis 120 mm aufweisen, vorzugsweise zwischen 80 mm +/- 10 mm, wobei alternativ oder ergänzend ein Innenwinkel alpha einer Innenfläche des Glockentellers z. B. zwischen 5° und 45° betragen kann, vorzugsweise zwischen 30° +/- 5°.

Ein Außenwinkel beta der Außenfläche des Glockentellers kann zwischen 1° und 80° liegen, vorzugsweise zwischen 30° +/- 5°.

Vorzugsweise ist der Innenwinkel alpha kleiner als der Außenwinkel beta.

Die Innenfläche des Glockentellers kann einen insbesondere ringförmigen Randbereich aufweisen, wobei der Randbereich zweckmäßig an die Glockentellerabspritzkante anschließt, eine Breite von z. B. zwischen 0,05 mm und 10 mm (vorzugsweise 2mm +/- 1mm) aufweisen kann und vorzugsweise nur der Randbereich mit einer Oberflächenstruktur versehen ist, wobei der verbleibende Teil der Innenfläche oder zumindest ein Großteil davon vorzugsweise ohne Oberflächenstruktur ausgeführt sein kann.

Im Kontext der Erfindung kann die hohle Antriebswelle z. B. ein hohles Innenrohr umfassen, wobei das Desinfektionsmittel über das hohle Innenrohr dem Glockenteller zugeführt werden kann.

Die Basis des Glockentellers dient vorzugsweise zur Montage, um den Glockenteller mit der hohlen Antriebswelle rotieren lassen zu können.

Die Innenfläche dient insbesondere zum Verteilen und/oder Transportieren des Desinfektionsmittels zur Glockentellerabspritzkante.

Ein Durchlassquerschnitt der hohlen Antriebswelle kann z. B. zwischen 3 mm und 30 mm liegen.

Die oben beschriebenen bevorzugten Ausführungsformen der Erfindung sind miteinander kombinierbar. Andere vorteilhafte Weiterbildungen der Erfindung sind in den Unteransprüchen offenbart oder ergeben sich aus der folgenden Beschreibung bevorzugter Ausführungsformen der Erfindung in Verbindung mit den beigefügten Figuren.
- Figur 1: zeigt eine schematische Ansicht einer Vorrichtung gemäß einer Ausführungsform der Erfindung,
- Figur 2: zeigt eine schematische Ansicht der Vorrichtung der Figur 1, insbesondere zur Erläuterung eines Desinfektionsprozesses-/modus, gemäß einer Ausführungsform der Erfindung,
- Figur 3: zeigt eine schematische Ansicht der Vorrichtung der Figur 1, insbesondere zur Erläuterung eines Verlustmittel-Rückgewinnungsprozesses-/modus, gemäß einer Ausführungsform der Erfindung,
- Figur 4: zeigt eine schematische Ansicht der Vorrichtung der Figur 1, insbesondere zur Erläuterung eines Spülprozesses-/modus, gemäß einer Ausführungsform der Erfindung,
- Figur 5: zeigt eine schematische Ansicht einer modifizierten Ausführungsform für die Vorrichtung der Figur 1 gemäß einer Ausführungsform der Erfindung,
- Figur 6: zeigt eine schematische Ansicht einer modifizierten Ausführungsform für die Vorrichtung der Figur 1 gemäß einer Ausführungsform der Erfindung,
- Figur 7: zeigt eine schematische Ansicht einer modifizierten Ausführungsform für die Vorrichtung der Figur 1 gemäß einer Ausführungsform der Erfindung,
- Figur 8: zeigt eine schematische Ansicht eines mobilen Transportgehäuses,
- Figur 9: zeigt eine Querschnittsansicht eines Glockentellers und anderer Komponenten gemäß einer Ausführungsform der Erfindung,
- Figur 10: zeigt eine Perspektivansicht von oben eines Glockentellers gemäß einer Ausführungsform der Erfindung, und
- Figur 11: zeigt eine Perspektivansicht von unten des Glockentellers der Figur 10.

Die unter Bezugnahme auf die Figuren beschriebenen bevorzugten Ausführungsformen der Erfindung stimmen teilweise überein, wobei ähnliche oder identische Teile mit den gleichen Bezugszeichen versehen sind, und zu deren Erläuterung auch auf die Beschreibung anderer Ausführungsformen oder Figuren verwiesen wird, um Wiederholungen zu vermeiden.

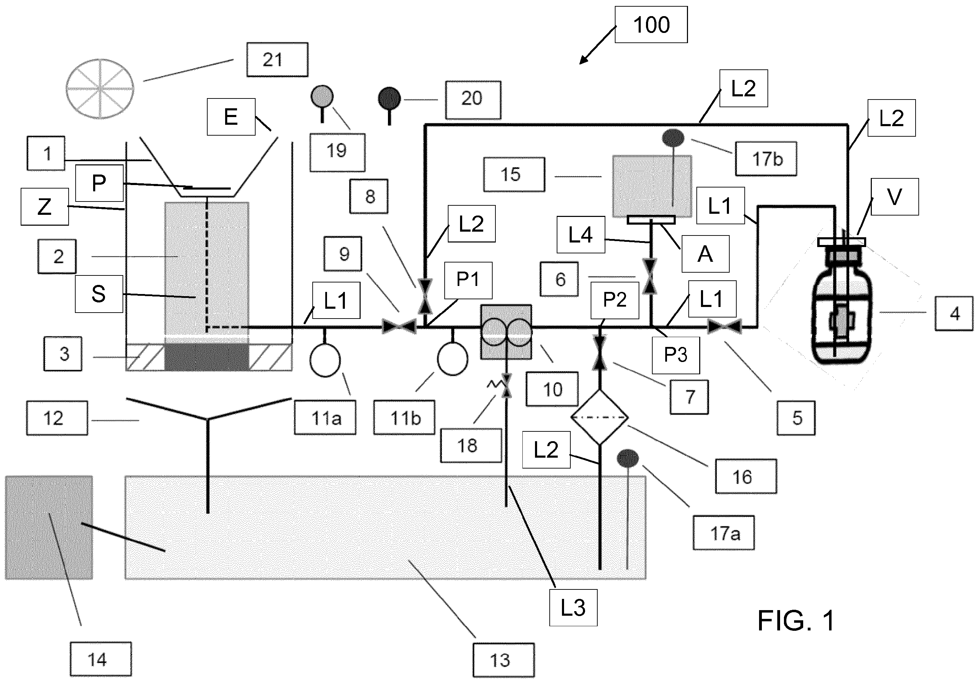

Figur 1 zeigt eine schematische Ansicht einer Vorrichtung 100 gemäß einer Ausführungsform der Erfindung zum Desinfizieren zumindest eines Raums, insbesondere eines Aufenthaltsraums für eine oder mehrere Personen. Der Aufenthaltsraum kann z. B. ein humanmedizinischer oder veterinärer Behandlungsraum sein, insbesondere ein Krankenzimmer, ein Patientenzimmer und/oder ein Operationssaal, z. B. eines Krankenhauses oder einer Arztpraxis.

Die Vorrichtung 100 umfasst einen Zerstäuber für ein Desinfektionsmittel zum Desinfizieren des Aufenthaltsraums und zeichnet sich z. B. dadurch aus, dass der Zerstäuber einen rotierbaren Glockenteller 1 zum Zerstäuben des Desinfektionsmittels in den Aufenthaltsraum umfasst.

Ein Elektromotor 2 oder eine Luftturbine ist vorgesehen, um den Glockenteller 1 anzutreiben und somit in Drehung zu versetzen und zwar mittels einer hohlen Antriebswelle S, die zugleich vorteilhaft zum Zuführen des Desinfektionsmittels zum Glockenteller 1 dient. Hierfür ist in der hohlen Antriebswelle S eine Zuleitung zur Verfügung gestellt. Der Glockenteller 1 ist zweckmäßig fest an der Antriebswelle S befestigt.

Der Glockenteller 1 wirkt mit einer scheibenförmigen Platte P zusammen, die zweckmäßig mit dem Glockenteller 1 mitrotiert. Der Glockenteller 1, die Antriebswelle S und die Platte P sind im Wesentlichen koaxial zueinander angeordnet.

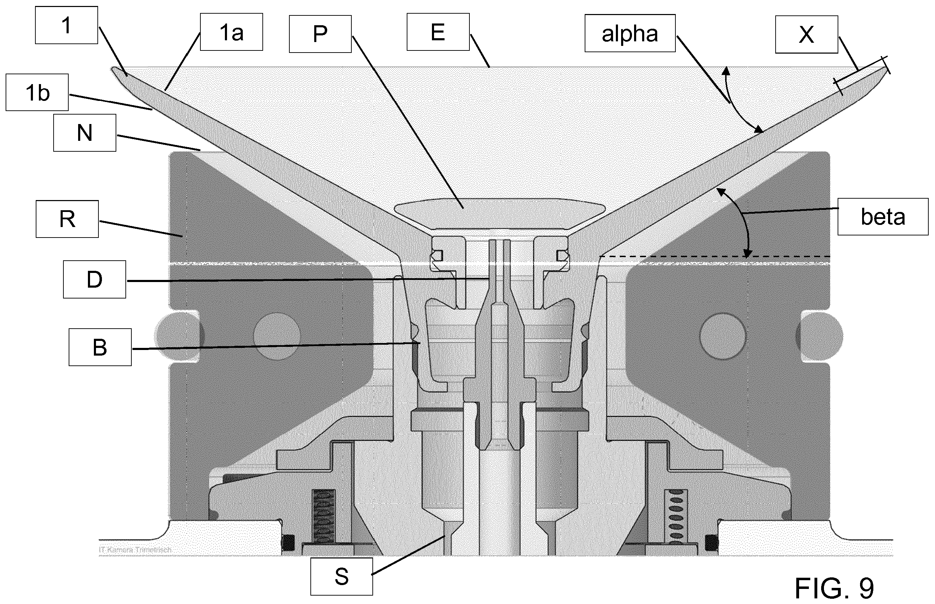

Das aus einer Desinfektionsmittel-Austrittsdüse D der Antriebswelle S austretende Desinfektionsmittel trifft auf die rotierende Platte P und wird dadurch Zentrifugalkraft-basiert auf eine Innenfläche 1a des rotierenden Glockentellers 1 verteilt (Figur 9), von wo aus das Desinfektionsmittel zu einer ringförmigen Glockentellerabspritzkante E geführt wird, von wo aus es zerstäubt und in den Aufenthaltsraum abgespritzt wird.

Der Glockenteller 1 weist im Betrieb eine Drehzahl von vorzugsweise mindestens 30.000 Umdrehungen pro Minute und eine Zerstäubungsleistung von mindestens 50 Milliliter pro Minute und/oder von mindestens 0,3 Kubikmeter pro Minute auf.

Glockenteller und deren Wirkweise insbesondere mit mitrotierender Platte sind im technischen Bereich der Lackierung von Kraftfahrzeugkarosserien mittels Rotationszerstäubern üblich, so dass hierauf für Einzelheiten verwiesen werden kann. Der Glockenteller dient dabei dazu, den Lack zu zerstäuben und in Form eines Lack-Sprühstrahls zur Erzeugung einer Lackbahn auf die Kraftfahrzeugkarosserien abzugeben.

Ein erstes Gebläse 3 ist zweckmäßig stromaufwärts des Glockentellers 1 angeordnet, wobei ein zweites, vorzugsweise als Seitengebläse ausgeführtes Gebläse 21, stromabwärts des Glockentellers 1 angeordnet ist. Die Gebläse 3, 21 dienen zum Transportieren und Verteilen von mittels des Glockentellers 1 zerstäubtem Desinfektionsmittel, so dass zerstäubtes Desinfektionsmittel möglichst optimal im Aufenthaltsraum verteilt werden kann. Dadurch kann gewährleistet werden, dass das Desinfektionsmittel möglichst alle zu desinfizierenden Flächen (z.B. Wände, Decken, Einrichtungsgegenstände, abgewandte Flächen, etc.) erreicht, um diese zu desinfizieren. Die Gebläse 3 und 21 können z. B. eine Gesamt-Luftleistung von über 200 oder sogar über 300 Kubikmeter pro Stunde aufweisen.

Der Glockenteller 1, der Elektromotor 2 oder die Luftturbine und das Gebläse 3 sind zumindest abschnittsweise in einem Zerstäubergehäuse Z untergebracht.

Eine die Antriebswelle S umfassende Desinfektionsmittel-Leitung L1 verbindet den Glockenteller 1 mit einer Verbindungseinrichtung V. Die Verbindungseinrichtung V dient zum Verbinden mit einem Behälter 4 zur Aufnahme des Desinfektionsmittels. Die Desinfektionsmittel-Leitung L1 dient also insbesondere zum Zuführen des Desinfektionsmittels 1 zum Glockenteller 1. Eine Fördereinrichtung 10, z. B. eine Pumpe, dient dabei zum Fördern und Dosieren des Desinfektionsmittels.

Die Vorrichtung 100 umfasst zwei Durchflussmesseinrichtungen 11a, 11b zum Messen eines Volumenstroms insbesondere des Desinfektionsmittels. Eine erste Durchflussmesseinrichtung 11a ist zwischen dem Glockenteller 1 und einem stromabwärts der Fördereinrichtung 10 angeordneten Glockenteller-Ventil 9 angeordnet. Eine zweite Durchflussmesseinrichtung 11b ist zwischen der Fördereinrichtung 10 und dem stromabwärts der Fördereinrichtung 10 angeordneten Glockenteller-Ventil 9 angeordnet.

Zumindest ein Luftentfeuchter 14 dient zur Entfeuchtung von Luft im Aufenthaltsraum, insbesondere nach Beendigung der Zerstäubung des Desinfektionsmittels. Der oder die Luftentfeuchter 14 weisen vorzugsweise eine Gesamt-Entfeuchtungsleistung von über 600 oder über 700 Kubikmeter pro Stunde auf.

Ein Aufnahmebehälter 13 z. B. in Form einer Wanne dient zur Aufnahme und Rückgewinnung von durch den Desinfektionsprozess entstehendem Verlustmittel.

Der Aufnahmebehälter 13 steht mit dem Zerstäubergehäuse Z über einen Trichter 12 in Verbindung, um Flüssigkeit aufnehmen zu können, die an oder in dem Zerstäubergehäuse Z abgeschieden wird, insbesondere aus dem Desinfektionsmittel abgeschieden wird.

Der Aufnahmebehälter 13 steht ferner mit dem Luftentfeuchter 14 in Verbindung, um Flüssigkeit aufnehmen zu können, die der Luftentfeuchter 14 der Raumluft entzieht.

Der Aufnahmebehälter 13 steht außerdem über eine Leitung L3 mit der Fördereinrichtung 10 in Verbindung, so dass Flüssigkeit dem Aufnahmebehälter 13 zugeführt werden kann, wenn an einem in der Leitung L3 angeordnetem Überdruckventil 18 Überdruck anliegt. Das Überdruckventil 18 kann alternativ auch in der Desinfektionsmittel-Leitung L1 z. B. zwischen Fördereinrichtung 10 und Glockenteller 1 angeordnet sein, insbesondere an oder neben der zweiten Durchflussmesseinrichtung 11b, oder an oder in der Fördereinrichtung 10.

Die Vorrichtung 100 umfasst eine Verlustmittel-Leitung L2, wobei der Aufnahmebehälter 13 über die Verlustmittel-Leitung L2 entleerbar ist und vorzugsweise mit der Verbindungseinrichtung V in Verbindung bringbar ist, so dass Verlustmittel aus dem Aufnahmebehälter 13 über die Verbindungseinrichtung V z. B. dem zuvor im Desinfektionsprozess entleerten Desinfektionsmittel-Behälter 4 oder einem anderen Behälter zur Entsorgung zuführbar ist.

Das Verlustmittel passiert auf seinem Weg vom Aufnahmebehälter 13 zur Verbindungseinrichtung V folgendes: eine Filtereinrichtung 16 zur Filterung des Verlustmittels, die Fördereinrichtung 10, ein Aufnahmebehälter-Ventil 7, das stromabwärts der Filtereinrichtung 16 und stromaufwärts der Fördereinrichtung 10 angeordnet ist, ein Verlustmittel-Rückström-Ventil 8, das stromabwärts der Fördereinrichtung 10 und stromaufwärts der Verbindungseinrichtung V angeordnet ist, und die Durchflussmesseinrichtung 11b.

Ein Spülmittelanschluss A dient zur Verbindung mit einem Spülmittel-Behälter 15 zur Aufnahme eines Spülmittels, um in einem Spülmodus zumindest abschnittsweise die Desinfektionsmittel-Leitung L1 und/oder die Verlustmittel-Leitung L2 zu spülen.

Der Spülmittelanschluss A ist über eine Spülmittel-Leitung L4 mit der Desinfektionsmittel-Leitung L1 in Verbindung bringbar, wobei die Spülmittel-Leitung L4 vor Mündung in die Desinfektionsmittel-Leitung L1 ein Spülmittel-Ventil 6 umfasst.

Die Vorrichtung 100 ist somit nicht nur in einem Desinfektionsmodus betreibbar, sondern ist ferner in einem Rückgewinnungs-Modus zum Rückgewinnen von mittels des Desinfektionsprozesses entstehendem Verlustmittel und in einem Spülmodus zum zumindest abschnittsweisen Spülen der Desinfektionsmittel-Leitung L1 und vorzugsweise der Verlustmittel-Leitung L2 betreibbar.

Die Fördereinrichtung 10 ist dabei universell verwendbar. So dient die Fördereinrichtung 10 im Desinfektionsmodus zum Fördern des Desinfektionsmittels aus dem Desinfektionsmittel-Behälter 4, im Rückgewinnungs-Modus zum Fördern des Verlustmittels aus dem Aufnahmebehälter 13 und im Spülmodus zum Fördern des Spülmittels aus dem Spülmittel-Behälter 15.

Die Verlustmittel-Leitung L2 schließt an zwei Knotenpunkten P1 und P2 an die Desinfektionsmittel-Leitung L1 an.

Der Knotenpunkt P1 ist zwischen der Fördereinrichtung 10 und dem in der Desinfektionsmittel-Leitung L1 stromabwärts der Fördereinrichtung 10 angeordneten Glockenteller-Ventil 9 angeordnet.

Der Knotenpunkt P2 ist zwischen der Fördereinrichtung 10 und einem in der Desinfektionsmittel-Leitung L1 stromaufwärts der Fördereinrichtung 10 angeordneten Desinfektionsmittel-Ventil 5 angeordnet.

Die Spülmittel-Leitung L4 schließt an einem Knotenpunkt P3 an die Desinfektionsmittel-Leitung L1 an, wobei der Knotenpunkt P3 zwischen der Fördereinrichtung 10 und dem in der Desinfektionsmittel-Leitung L1 stromaufwärts der Fördereinrichtung 10 angeordneten Desinfektionsmittel-Ventil 5 angeordnet ist.

Das Desinfektionsmittel passiert im Desinfektionsprozess folgendes: das Desinfektionsmittel-Ventil 5 stromaufwärts der Fördereinrichtung 10, das Glockenteller-Ventil 9 stromabwärts der Fördereinrichtung 10, die Fördereinrichtung 10, den Elektromotor 2 oder die Luftturbine, die hohle Antriebswelle S mit der Desinfektionsmittel-Austrittsdüse D und die zwei Durchflussmesseinrichtungen 11a, 11b, die Platte P und den Glockenteller 1.

Das Spülmittel passiert im Spülmodus folgendes: die Fördereinrichtung 10, einen Teilabschnitt der Desinfektionsmittel-Leitung L1, die hohle Antriebswelle S mit Desinfektionsmittel-Austrittsdüse D, einen Teilabschnitt der Verlustmittel-Leitung L2, die Durchflussmesseinrichtungen 11a, 11b, das in der Desinfektionsmittel-Leitung L1 stromabwärts der Fördereinrichtung 10 angeordnete Glockenteller-Ventil 9, den Elektromotor 2 oder die Luftturbine, den Glockenteller 1, die Platte P, das in der Verlustmittel-Leitung L2 stromabwärts der Fördereinrichtung 10 angeordnete Verlustmittel-Rückström-Ventil 8 und die Verbindungseinrichtung V.

Eine Feuchtigkeitsmesseinrichtung 19 z. B. mit Regelwirkung auf den Desinfizierungsprozess der Vorrichtung 100 ist vorgesehen, wobei die Feuchtigkeitsmesseinrichtung 19 zum Messen einer Feuchtigkeit in dem Aufenthaltsraum dient. So kann z. B. der Desinfektionsprozess gestoppt werden, wenn eine mangelnde Zunahme der Feuchte im Aufenthaltsraum während des Zerstäubungsvorgangs erkannt wird.

Eine Temperaturmesseinrichtung 20 z. B. mit Regelwirkung auf den Desinfizierungsprozess der Vorrichtung 100 ist vorgesehen, wobei die Temperaturmesseinrichtung 20 zum Messen einer Temperatur im Aufenthaltsraum dient.

Eine Füllstandsmesseinrichtung 17a dient zum Messen eines in dem Aufnahmebehälter 13 vorliegenden Füllstands, wobei eine Füllstandsmesseinrichtung 17b zum Messen eines in dem Spülmittel-Behälter 15 vorliegenden Füllstands dient. Ebenso kann z. B. eine Füllstandsmesseinrichtung zum Messen eines in dem Desinfektionsmittel-Behälter 4 vorliegenden Füllstands dienen.

Eine nicht dargestellte Raumvermessungseinrichtung dient zum Vermessen des Aufenthaltsraums, wobei in Abhängigkeit von Messdaten der Raumvermessungseinrichtung der Desinfizierungsprozess der Vorrichtung 100 erfolgt (z. B. Anpassung der Dosiermenge des Desinfektionsmittels, des Volumenstroms des zumindest einen Gebläses 3, 21 und/oder der Prozesszeit, etc.) oder aber eine Einleitung eines Desinfizierungsprozesses der Vorrichtung 100 unterbunden wird, z. B. wenn durch die Raumvermessungsreinrichtung festgestellt wird, dass die Vorrichtung 100 unpassend im Aufenthaltsraum positioniert ist oder der Aufenthaltsraum zum Desinfizieren durch die Vorrichtung 100 ungeeignet ist.

Die Vorrichtung 100 kann außerdem eine oder mehrere nicht dargestellte Erkennungseinrichtungen umfassen, zum Erkennen des Desinfektionsmittels, zum Erkennen des Spülmittels und/oder zum Erkennen einer Drehzahl des Glockentellers 1. Die Vorrichtung 100 kann z. B. konfiguriert sein, um ihren Betrieb zu unterbrechen oder zu unterbinden (z. B. zu stoppen oder erst gar nicht zu starten), wenn die Erkennungseinrichtung ein unpassendes Desinfektionsmittel, ein unpassendes Spülmittel und/oder eine unpassende Drehzahl des Glockentellers 1 erkennt.

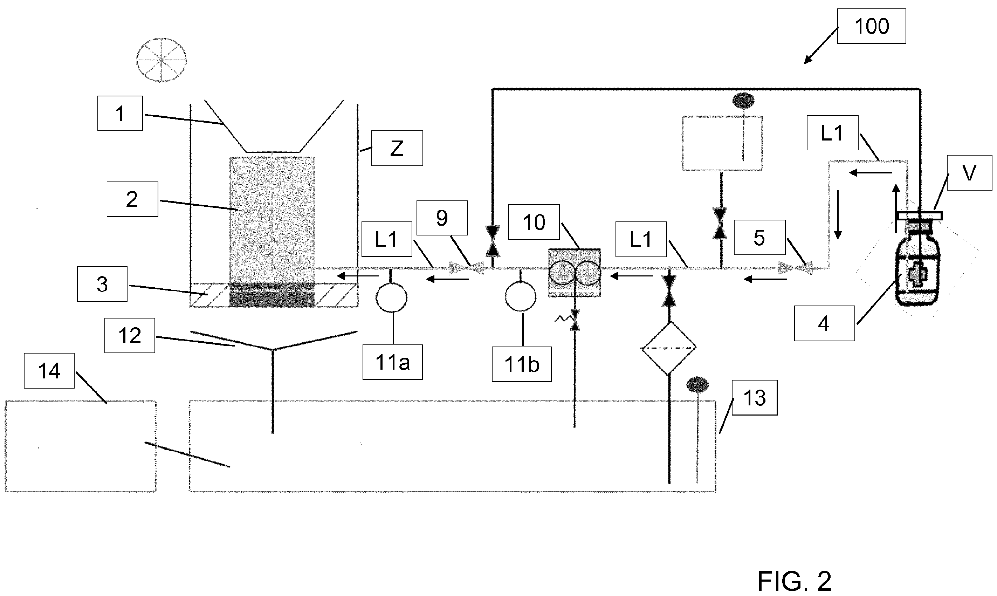

Figur 2 zeigt eine schematische Ansicht der Vorrichtung 100, insbesondere zur Erläuterung eines Desinfektionsprozesses-/modus gemäß einer Ausführungsform der Erfindung.

Der Desinfektionsprozess funktioniert im Wesentlichen wie folgt:
Der Elektromotor 2, das Gebläse 3 und die Temperaturmesseinrichtung 20 werden aktiviert und arbeiten basierend auf Prozesswerten für den Desinfizierungsprozess.

Das Desinfektionsmittel-Ventil 5 und das Glockenteller-Ventil 9 in der Desinfektionsmittel-Leitung L1 werden geöffnet. Die Fördereinrichtung 10 wird aktiviert, so dass das Desinfektionsmittel aus dem Desinfektionsmittel-Behälter 4 gefördert wird.

Nach einer vordefinierten Zeitspanne von x Sekunden erfassen die Durchflussmesseinrichtungen 11a und 11b eine Volumenströmung des Desinfektionsmittels. Wenn keine oder eine zu kleine Volumenströmung erfasst wird, wird der Desinfizierungsprozess sofort gestoppt.

Das Gebläse 3 zieht Luft aus der Umgebung und nutzt sie, um zerstäubtes Desinfektionsmittel im Aufenthaltsraum zu verteilen

Das Überdruckventil 18 schützt die Vorrichtung 100 vor zu hohem Überdruck.

Der Trichter 12 sammelt Feuchtigkeit, welche sich eventuell an oder in dem Zerstäubergehäuse Z während des Desinfizierungsprozesses abscheidet, und leitet sie zum Aufnahmebehälter 13.

Nach einer vorbestimmten Zeitspanne (Volumen des Desinfektionsmittel-Behälters 4 / Volumenströmung) und wenn die Durchflussmesseinrichtung 11a keine Strömung erfasst, ist der Desinfizierungsprozess beendet. Somit hört die Fördereinrichtung 10 auf, zu laufen. Das Desinfektionsmittel-Ventil 5 und das Glockenteller-Ventil 9 in der Desinfektionsmittelleitung L1 werden geschlossen. Der Elektromotor 2 wird ausgeschaltet. Das Gebläse 3 ändert zweckmäßig seine Funktionsweise und arbeitet z. B. basierend auf Prozesswerten für einen Verweilprozess und/oder zum Kühlen des Elektromotors 2.

Durch den Desinfektionsprozess kann in dem Aufenthaltsraum eine relative Raumfeuchte von z. B. zwischen 85 % bis 99% erzeugt werden.

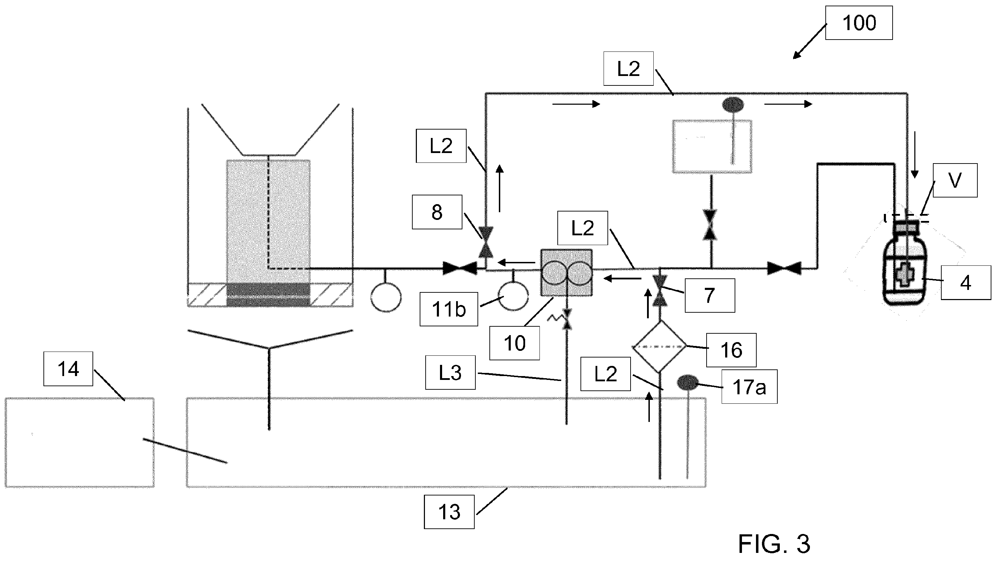

Figur 3 zeigt eine schematische Ansicht der Vorrichtung 100, insbesondere zur Erläuterung eines Verlustmittel-Rückgewinnungsprozesses-/modus, gemäß einer Ausführungsform der Erfindung.

Der Verlustmittel-Rückgewinnungsprozess funktioniert im Wesentlichen wie folgt:
Der Verlustmittel-Rückgewinnungsprozess startet eine vorbestimmte Zeitspanne vor Beendigung des Entlüftungsvorgangs des Luftentfeuchters 14.

Abgeschiedene Flüssigkeit aus dem Zerstäubergehäuse Z, Wasser aus dem Luftentfeuchter 14 und eventuell Flüssigkeit aus der Leitung L3 (nur im Fall von Überdruck im Leitungssystem der Vorrichtung 100) werden dem Aufnahmebehälter 13 als Verlustmittel zugeführt.

Das Aufnahmebehälter-Ventil 7 und das Verlustmittel-Rückström-Ventil 8 in der Verlustmittel-Leitung L2 werden geöffnet. Die Fördereinrichtung 10 beginnt, das Verlustmittel aus dem Aufnahmebehälter 13 zum zuvor entleerten Desinfektionsmittel-Behälter 4 zu fördern oder aber zu einem anderen Behälter.

Die Filtereinrichtung 16 verhindert das Eindringen fester Partikel oder allgemein unerwünschter Teile in das Leitungssystem der Vorrichtung 100.

Wenn die Durchflussmesseinrichtung 11b erfasst, dass keine Verlustmittelströmung vorliegt, ist der Verlustmittel-Rückgewinnungsprozess beendet. Zu erwähnen ist, dass der Verlustmittel-Rückgewinnungsprozess vorzugsweise innerhalb der Zeitspanne beendet ist, die für das Entfeuchten des Aufenthaltsraums durch den Luftentfeuchter 14 erforderlich ist.

Die Fördereinrichtung 10 hört auf, zu laufen. Das Aufnahmebehälter-Ventil 7 und das Verlustmittel-Rückström-Ventil 8 in der Verlustmittel-Leitung L2 werden geschlossen.

Eine kleine Menge an Verlustmittel kann im Aufnahmebehälter 13 verbleiben.

Die Füllstandsmesseinrichtung 17a prüft, ob ein sachgemäßer Füllstand im Aufnahmebehälter 13 vorliegt. Daraus kann gefolgert werden, ob der Verlustmittel-Rückgewinnungsprozess sachgemäß erfolgt ist oder nicht.

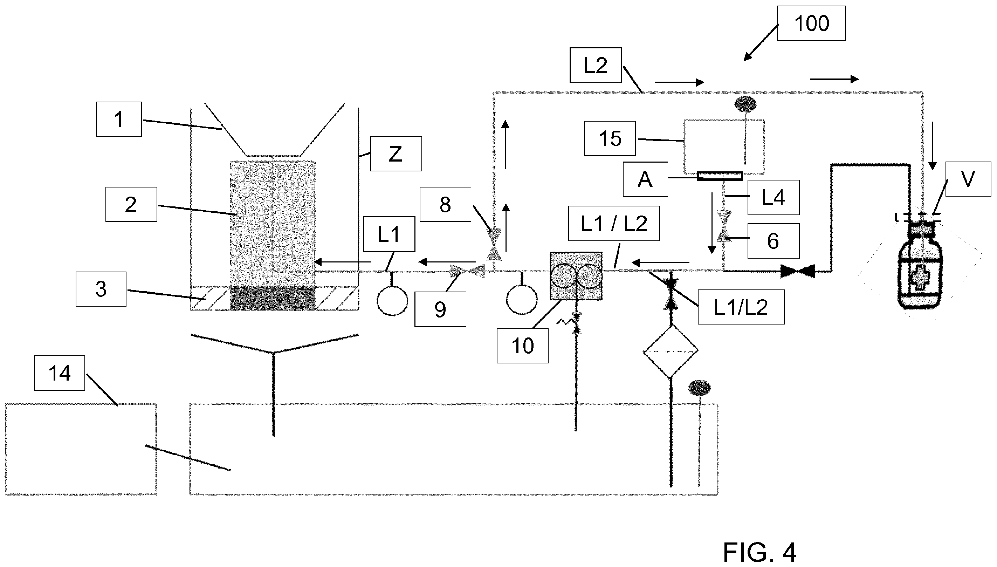

Figur 4 zeigt eine schematische Ansicht der Vorrichtung 100, insbesondere zur Erläuterung eines Spülprozesses-/modus gemäß einer Ausführungsform der Erfindung

Der Spülprozess funktioniert im Wesentlichen wie folgt:
Der Spülprozess startet nach Beendigung der Luftentfeuchtung durch den Luftentfeuchter 14.

Der Elektromotor 2 und das Gebläse 3 starten und arbeiten basierend auf Prozesswerten für den Spülprozess.

Das Spülmittel-Ventil 6, das Verlustmittel-Rückström-Ventil 8 und das Glockenteller-Ventil 9 werden geöffnet. Die Fördereinrichtung 10 beginnt, Spülmittel aus dem Spülmittel-Behälter 15 zu fördern.

Das Spülmittel wird teilweise zum Glockenteller 1 und teilweise zurück zum Desinfektionsmittel-Behälter 4 gefördert oder aber zu einem anderen Behälter.

Der Spülmittelprozess wird nach einer vordefinierten Volumenströmung beendet, z. B. nach einer vordefinierten Laufzeit der Fördereinrichtung 10. Die Fördereinrichtung 10 hört auf, zu laufen. Das Spülmittel-Ventil 6, das Verlustmittel-Rückström-Ventil 8 und das Glockenteller-Ventil 9 werden geschlossen. Der Elektromotor 2 und das Gebläse 3 hören auf, zu laufen.

Eine kleine Menge an Spülmittel kann im Leitungssystem verbleiben.

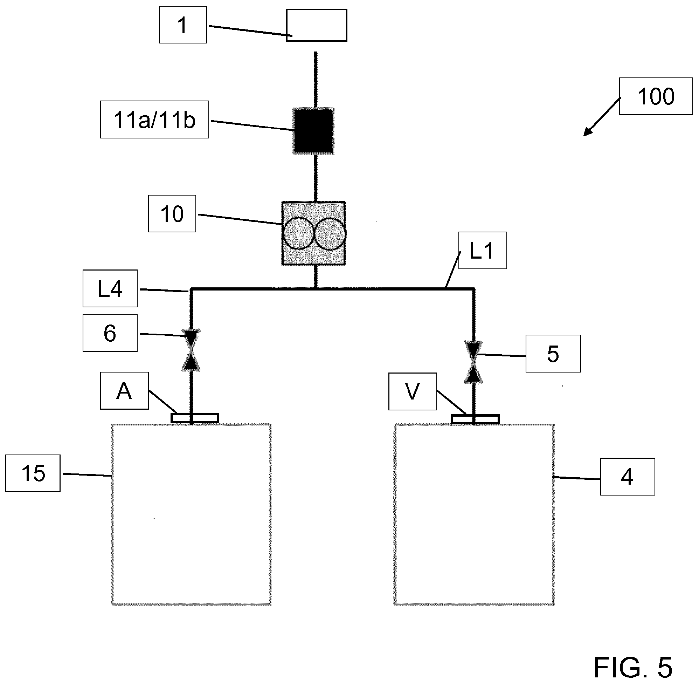

Figur 5 zeigt eine schematische Ansicht einer modifizierten Ausführungsform für die Vorrichtung 100 gemäß einer Ausführungsform der Erfindung.

Eine Besonderheit bei der in Figur 5 gezeigten Ausführungsform ist, dass an den Spülmittelanschluss A ein fest fixierter Spülmittel-Behälter 15 in Form eines nachfüllbaren Tanks anmontiert ist und an die Verbindungseinrichtung V ein fest fixierter Desinfektionsmittel-Behälter 4 in Form eines nachfüllbaren Tanks anmontiert ist.

Somit können im Kontext der Erfindung die Verbindungseinrichtung V und der Spülmittelanschluss A zweckmäßig als unlösbare oder lösbare Verbindungen ausgeführt sein.

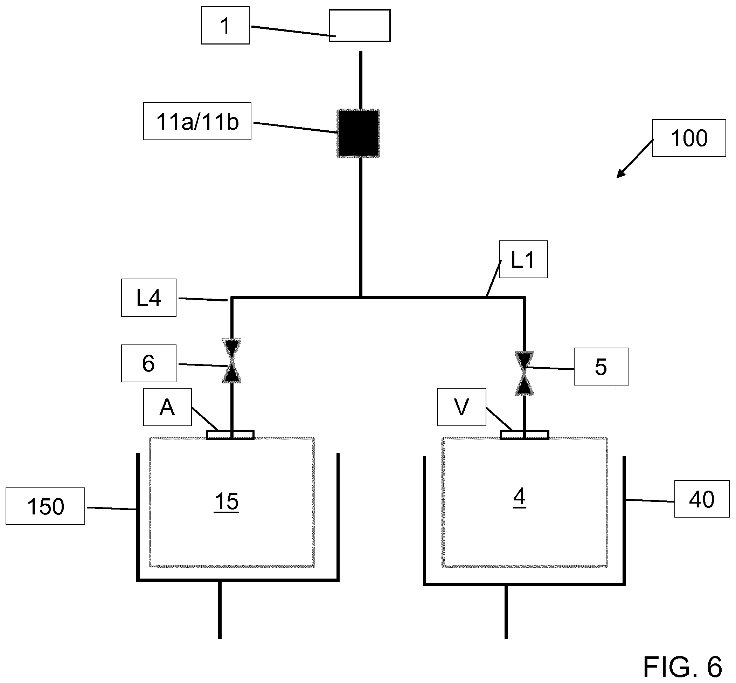

Figur 6 zeigt eine schematische Ansicht einer modifizierten Ausführungsform für die Vorrichtung 100 gemäß einer Ausführungsform der Erfindung.

Eine Besonderheit bei der in Figur 6 gezeigten Ausführungsform ist, dass die Fördereinrichtung ein Kolbensystem 40 zum Fördern des Desinfektionsmittels aus dem Desinfektionsmittel-Behälter 4 aufweist und ein Kolbensystem 150 zum Fördern des Spülmittels aus dem Spülmittel-Behälter 15 aufweist. Folglich liegen zwei Fördereinrichtungen (Kolbensysteme) 40 und 150 vor. Besonders bevorzugt ist dabei, wenn der Desinfektionsmittel-Behälter 4 als Einwegbehälter zur Aufnahme im Kolbensystem 40 ausgeführt ist und/oder der Spülmittel-Behälter 15 als Einwegbehälter zur Aufnahme im Kolbensystem 150 ausgeführt ist.

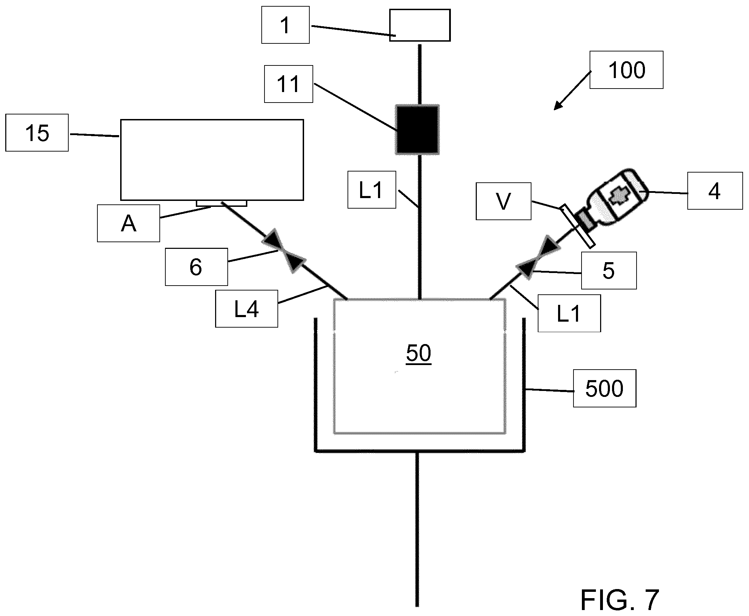

Figur 7 zeigt eine schematische Ansicht einer modifizierten Ausführungsform für die Vorrichtung 100 gemäß einer Ausführungsform der Erfindung.

Eine Besonderheit bei der in Figur 7 gezeigten Ausführungsform ist, dass nicht zwei separate Behälter 4 und 15 für das Desinfektionsmittel und das Spülmittel vorgesehen sind, sondern ein Multi-Behälter 50 zur Aufnahme des Desinfektionsmittels für den Desinfektionsprozess und zusätzlich zur Aufnahme von Spülmittel für den Spülmodus, so dass der Multi-Behälter 50 zunächst für den Desinfektionsprozess mit dem Desinfektionsmittel und danach für den Spülmodus mit dem Spülmittel befüllbar und betreibbar ist. Die Fördereinrichtung ist als Kolbensystem 500 ausgeführt, mit im Kolbensystem 500 integriertem Multi-Behälter 50. Der Multi-Behälter 50 ist in die Desinfektionsmittel-Leitung L1 und die Spülmittel-Leitung L4 integriert und ist über das Desinfektionsmittel-Ventil 5 mit der Verbindungseinrichtung V in Verbindung bringbar und über das Spülmittel-Ventil 6 mit dem Spülmittelanschluss A in Verindung bringbar.

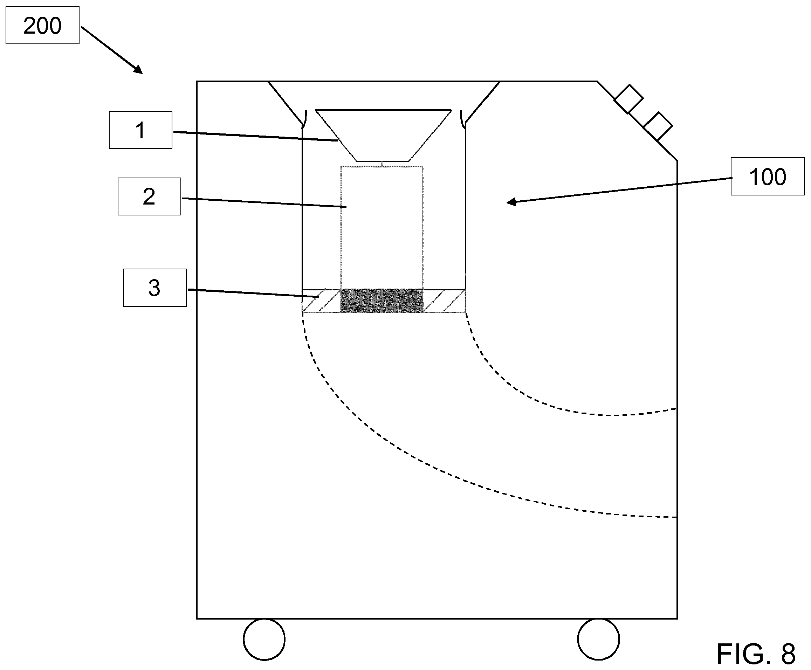

Figur 8 zeigt eine schematische Ansicht eines mobilen Transportgehäuses 200 mit aufgenommener Vorrichtung 100.

Das Transportgehäuse 200 mit der Vorrichtung 100 kann durch eine Bedienperson in einen Aufenthaltsraum geschoben werden. Die Bedienperson kann dann die Vorrichtung 100 aktivieren und daraufhin den Aufenthaltsraum verlassen. Nach Beendigung des Desinfektionsprozesses kann die Bedienperson dann das Transportgehäuse 200 mit der Vorrichtung 100 in einen anderen zu desinfizierenden Aufenthaltsraum transportieren. Hierzu ist das Transportgehäuse 200 Teil eines mit Rollen versehenen Transportwagens.

Das Transportgehäuse 200 legt den Glockenteller 1 zum Zerstäuben des Desinfektionsmittels zumindest abschnittsweise frei, wobei der Aufnahmebehälter 13 unter dem Niveau des Glockentellers 1 und unter dem Niveau des Gebläses 3 positioniert ist, so dass Verlustmittel Schwerkraft-basiert dem Aufnahmebehälter 13 zuführbar ist. Das Transportgehäuse 200 ist mit schalldämmendem Material versehen ist und umfasst eine Dichtungsplatte, um einen Innenraum des Transportgehäuses 200 in eine Trockensektion und eine Nasssektion zu unterteilen.

Figur 9 zeigt eine Querschnittsansicht eines Glockentellers 1 und anderer Komponenten gemäß einer Ausführungsform der Erfindung.

Der Glockenteller 1 ist im Wesentlichen trichterförmig ausgebildet, mit einer Basis B (z. B. einem Sockelabschnitt), einer Innenfläche 1a für das Desinfektionsmittel, einer Außenfläche 1b und einer ringförmigen Glockentellerabspritzkante E. Der Glockenteller 1 wirkt mit einer Platte P zusammen. Bezugszeichen R kennzeichnet einen Gasleitring mit mehreren Leckageöffnungen N. Das Gas ist vorzugsweise Luft.

Die Innenfläche 1a kann geradlinig, konkav oder konvex ausgeformt sein.

Die Außenfläche 1b kann geradlinig, konkav oder konvex ausgeformt sein.

Die Basis B ist relativ zur Antriebswelle S drehfest montiert, so dass der Glockenteller 1 durch die Antriebswelle S antreibbar ist. Die Platte P ist drehfest an den Glockenteller 1 montiert, um mit dem Glockenteller 1 mitzudrehen. Die hohle Antriebswelle S umfasst ein hohles Innenrohr, über das das Desinfektionsmittel dem Glockenteller 1 zugeführt werden kann, wobei die Desinfektionsmittel-Austrittsdüse D zweckmäßig an das Innenrohr montiert sein kann.

Die ringförmige Glockentellerabspritzkante E kann z. B. einen Durchmesser von zwischen 65 mm bis 120 mm aufweisen, vorzugsweise zwischen 80 mm +/- 10 mm, wobei ein Innenwinkel alpha der Innenfläche 1a des Glockentellers 1 z. B. zwischen 5° und 45° betragen kann, vorzugsweise zwischen 30° +/- 5°. Ein Außenwinkel beta der Außenfläche 1b des Glockentellers 1 kann zwischen 1° und 80° liegen. Vorzugsweise ist der Innenwinkel alpha kleiner als der Außenwinkel beta.

Die Innenfläche 1a des Glockentellers 1 kann einen insbesondere ringförmigen Randbereich aufweisen, wobei der Randbereich an die Glockentellerabspritzkante E anschließt, eine Breite X von z. B. zwischen 0,05 mm und 10 mm (vorzugsweise 2mm +/- 1mm) aufweist und vorzugsweise nur der Randbereich mit einer Oberflächenstruktur versehen ist, nicht jedoch der verbleibende Teil der Innenfläche 1a.

Ein Durchlassquerschnitt für das Desinfektionsmittel der hohlen Antriebswelle S kann z. B. zwischen 3 mm und 30 mm liegen.

Im Desinfektionsprozess der Vorrichtung 100 trifft das Desinfektionsmittel, das aus der Desinfektionsmittel-Austrittsdüse D austritt, auf die rotierende Platte P, wird von der rotierenden Platte P auf die rotierende Innenfläche 1a verteilt und gelangt über die rotierende Innenfläche 1a zur rotierenden Glockentellerabspritzkante E, von wo aus das Desinfektionsmittel zerstäubt und in den Aufenthaltsraum abgegeben wird.

Der Gasleitring R mit den Leckageöffnungen N ist so angeordnet, dass aus den Leckageöffnungen N ausgegebenes Gas über die Außenfläche 1b des Glockentellers 1 auf das vom Glockenteller 1 abgegebene Desinfektionsmittel trifft, um das Desinfektionsmittel zu formen und/oder dessen Tröpfchengröße zu reduzieren.

Der Desinfektionsprozess läuft im Wesentlichen wie folgt ab:
Das Desinfektionsmittel wird durch die Antriebswelle S geführt und aus der Desinfektionsmittel-Austrittsdüse D der Antriebswelle S ausgespritzt, trifft in definiertem Abstand zur Platte P auf die Platte P und wird infolge der Zentrifugalkraft durch einen definierten Spalt zwischen dem Glockenteller 1 und der Platte P auf die Innenfläche 1a des Glockentellers 1a geleitet. Durch die spezielle Geometrie der Desinfektionsmittel-Austrittsdüse D und deren definierten Abstand zur Platte P wird gewährleistet, dass das Desinfektionsmittel vollständig dem definierten Spalt zugeführt wird. Dadurch wird eine homogen Verteilung auf der Innenfläche 1a gewährleistet. Durch die Zentrifugalkraft, infolge der Rotation, wird das Desinfektionsmittel entlang der speziell angestellten Innenflächen 1a in Richtung der Glockentellerabspritzkante E geleitet. An der Glockentellerabspritzkante E wird das Desinfektionsmittel in feinste Tröpfchen zerstäubt. Infolge der speziellen Geometrie der Glockentellerabspritzkante E und im Zusammenspiel mit der Formgasführung aus dem Gasleitring R wird der beschriebene Vorgang verbessert, insbesondere die Tröpfchengröße reduziert.

Durch die spezielle Geometrie der Außenfläche 1b und des Radius der Glockentellerabspritzkante E wird die Führung des Gases verbessert. Das Formgas wird vorzugsweise durch einen Lüfter bereitgestellt, läuft aufwärtsgerichtet entlang der Antriebswelle S und umströmt die Außenfläche 1b. Durch die spezielle Geometrie des Gasleitrings R wird das Formgas im Wesentlichen verwirbelungsfrei auf die Außenfläche 1b geleitet. Zudem erfüllt der Gasleitring R die Funktion der Geräuschminderung durch Vermeidung von Luftwirbelbildung im unteren Bereich der Außenfläche 1b, welcher vom Gasleitring R abgedeckt wird.

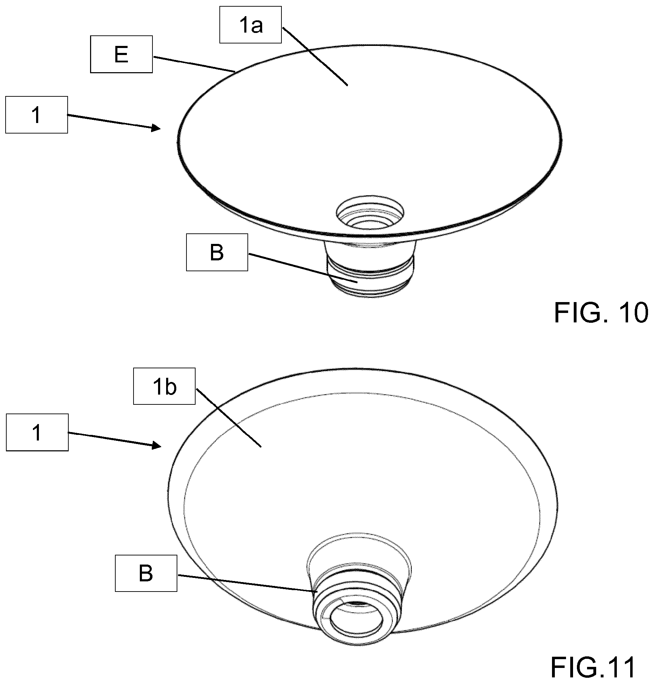

Die Figuren 10 und 11 zeigen zwei unterschiedliche Perspektivansichten eines Glockentellers 1 gemäß einer Ausführungsform der Erfindung. Der Glockenteller 1 dient zum Zerstäuben eines Desinfektionsmittels zum Desinfizieren zumindest eines Aufenthaltsraums für eine oder mehrere Personen und kann wie hierin offenbart ausgeführt sein.

Zu erwähnen ist noch, dass als Desinfektionsmittel z. B. das in US 2014/0119992 A1 offenbarte Desinfektionsmittel genutzt werden kann und/oder andere geeignete Desinfektionsmittel, um insbesondere Bakterien, Viren, Keime und/oder Pilze, etc. zu beseitigen. Das Desinfektionsmittel ist insbesondere ein flüssiges Desinfektionsmittel.

Zu erwähnen ist auch, dass es sich bei dem Raum vorzugsweise um einen durch eine oder mehrere Personen begehbaren Raum handeln kann.

### Bezugszeichenliste

- 1: Glockenteller
- 1a: Innenfläche des Glockentellers
- 1b: Außenfläche des Glockentellers
- X: Breite des Randbereichs der Innenfläche
- Alpha: Innenwinkel der Innenfläche
- Beta: Außenwinkel der Außenfläche
- B: Basis des Glockentellers, insbesondere Sockelabschnitt
- E: Abspritzkante des Glockentellers
- 2: Elektromotor
- 3: Gebläse
- 4: Behälter für Desinfektionsmittel
- 5: Insbesondere steuerbares Desinfektionsmittel-Ventil
- 6: Insbesondere steuerbares Spülmittel-Ventil
- 7: Insbesondere steuerbares Aufnahmebehälter-Ventil
- 8: Insbesondere steuerbares Verlustmittel-Rückström-Ventil
- 9: Glockenteller-Ventil
- 10: Fördereinrichtung, insbesondere Pumpe oder Kolbensystem
- 11a: Durchflussmesseinrichtung
- 11b: Durchflussmesseinrichtung
- 12: Trichter
- 13: Aufnahmebehälter für Verlustmittel
- 14: Luftentfeuchter
- 15: Behälter für Spülmittel
- 16: Filtereinrichtung
- 17a: Füllstandsmesseinrichtung für Aufnahmebehälter
- 17b: Füllstandsmesseinrichtung für Behälter für Spülmittel
- 18: Überdruckventil
- 19: Feuchtemesseinrichtung
- 20: Temperaturmesseinrichtung
- 21: Gebläse
- 40: Förderreinrichtung, insbesondere Kolbensystem
- 50: Multi-Behälter
- 150: Förderreinrichtung, insbesondere Kolbensystem
- 500: Förderreinrichtung, insbesondere Kolbensystem
- S: hohle Antriebswelle, vorzugsweise mit innerer Zuleitung für Desinfektinsmittel
- P: Platte, insbesondere Verteiler-/Prallplatte
- L1: Desinfektionsmittel-Leitung
- L2: Verlustmittel-Rückführ-Leitung
- L3: Leitung
- L4: Spülmittel-Leitung
- A: Spülmittelanschluss, zweckmäßig lösbar oder unlösbar
- V: Verbindungseinrichtung, zweckmäßig lösbar oder unlösbar
- D: Desinfektionsmittel-Austrittsdüse
- P1: Knotenpunkt
- P2: Knotenpunkt
- P3: Knotenpunkt
- R: Gasleitring, insbesondere Luftleitring
- N: Leckageöffnung(en), z. B. Gasaustrittsöffnung(en), insbesondere Luftaustrittsöffnung(en)
- Z: Zerstäubergehäuse
- 100: Vorrichtung

## Patentansprüche

1. Vorrichtung (100) zum Desinfizieren zumindest eines Raums, insbesondere für eine oder mehrere Personen, mit
a) einem Zerstäuber, der einen rotierbaren Glockenteller (1) zum Zerstäuben eines Desinfektionsmittels in den Raum umfasst,
b) einer Desinfektionsmittel-Leitung (L1), die mit dem Glockenteller (1) in Verbindung bringbar ist, um dem Glockenteller (1) das Desinfektionsmittel zuzuführen, und
c) einer Verbindungseinrichtung (V) zum Verbinden mit einem Desinfektionsmittel-Behälter (4) zur Aufnahme des Desinfektionsmittels, wobei die Verbindungseinrichtung (V) über die Desinfektionsmittel-Leitung (L1) mit dem Glockenteller (1) in Verbindung bringbar ist,
d) wobei die Vorrichtung (100) einen Aufnahmebehälter (13) zur Aufnahme von durch den Desinfektionsprozess entstehendem Verlustmittel aufweist,
e) wobei die Vorrichtung (100) eine Verlustmittel-Leitung (L2) aufweist,
f) wobei der Aufnahmebehälter (13) über die Verlustmittel-Leitung (L2) entleerbar ist, **dadurch gekennzeichnet,**
g) **dass** der Aufnahmebehälter (13) mit der Verbindungseinrichtung (V) in Verbindung bringbar ist, und
h) **dass** Verlustmittel aus dem Aufnahmebehälter (13) über die Verbindungseinrichtung (V) dem zuvor im Desinfektionsprozess entleerten Desinfektionsmittel-Behälter (4) zur Entsorgung zuführbar ist.

2. Vorrichtung (100) nach Anspruch 1, **dadurch gekennzeichnet, dass** die Vorrichtung (100) einen Elektromotor (2) und/oder eine Luftturbine zum Antreiben des Glockentellers (1) aufweist.

3. Vorrichtung (100) nach Anspruch 1 oder 2, **dadurch gekennzeichnet, dass** die Vorrichtung (100) zumindest ein Gebläse (3, 21) aufweist, wobei das Gebläse (3, 21) zum Transportieren und Verteilen von mittels des Glockentellers (1) zerstäubtem Desinfektionsmittel dient.

4. Vorrichtung (100) nach einem der vorhergehenden Ansprüche, **dadurch gekennzeichnet, dass** der Glockenteller (1), der Elektromotor (2), die Luftturbine und/oder das Gebläse (3) zumindest abschnittsweise in einem Zerstäubergehäuse (Z) untergebracht ist.

5. Vorrichtung (100) nach einem der vorhergehenden Ansprüche, **dadurch gekennzeichnet, dass** die Vorrichtung (100) zumindest eine Fördereinrichtung (10, 40, 150, 500) zum Fördern und/oder Dosieren des Desinfektionsmittels für den Desinfektionsprozess aufweist.

6. Vorrichtung (100) nach einem der vorhergehenden Ansprüche, **dadurch gekennzeichnet, dass** die Vorrichtung (100) zumindest eine Durchflussmesseinrichtung (11a, 11b) zum Messen eines Volumenstroms aufweist, wobei vorzugsweise.
- eine Durchflussmesseinrichtung (11a) zwischen dem Glockenteller (1) und einem stromabwärts der Fördereinrichtung (10, 40, 150, 500) angeordneten Glockenteller-Ventil (9) angeordnet ist, und/oder
- eine Durchflussmesseinrichtung (11b) zwischen der Fördereinrichtung (10, 40, 150, 500) und einem stromabwärts der Fördereinrichtung (10, 40, 150, 500) angeordneten Glockenteller-Ventil (9) angeordnet ist.

7. Vorrichtung (100) nach Anspruch 1, **dadurch gekennzeichnet, dass** der Aufnahmebehälter (13) zur Aufnahme von an oder in dem Zerstäubergehäuse (Z) aus dem Desinfektionsmittel abgeschiedener Flüssigkeit dient, wobei vorzugsweise die abgeschiedene Flüssigkeit über einen Trichter (12) dem Aufnahmebehälter (13) zuführbar ist.

8. Vorrichtung (100) nach einem der vorhergehenden Ansprüche, **dadurch gekennzeichnet, dass** die Vorrichtung (100) zumindest einen Luftentfeuchter (14) zur Entfeuchtung von Luft in dem Raum aufweist, mit vorzugsweise einer Gesamt-Entfeuchtungsleistung von über 600 oder über 700 Kubikmeter pro Stunde.

9. Vorrichtung (100) nach Anspruch 8, **dadurch gekennzeichnet, dass** der zumindest eine Luftentfeuchter (14) mit dem Aufnahmebehälter (13) in Verbindung bringbar ist, um aus der Luft entzogene Feuchtigkeit dem Aufnahmebehälter (13) zuzuführen.

10. Vorrichtung (100) nach einem der vorhergehenden Ansprüche, **dadurch gekennzeichnet, dass** die Fördereinrichtung (10, 40, 150, 500) über eine Leitung (L3) mit dem Aufnahmebehälter (13) in Verbindung bringbar ist, um über die Leitung (L3) Flüssigkeit dem Aufnahmebehälter (13) zuzuführen, wenn an einem Überdruckventil (18) Überdruck anliegt.

11. Vorrichtung (100) nach Anspruch 1, **dadurch gekennzeichnet, dass** der Weg des Verlustmittels vom Aufnahmebehälter (13) zur Verbindungseinrichtung (V) zumindest eines von Folgendem umfasst:
- eine Filtereinrichtung (16) zur Filterung des Verlustmittels,
- die Fördereinrichtung (10, 40, 150, 500),
- ein Aufnahmebehälter-Ventil (7), das vorzugsweise stromabwärts der Filtereinrichtung (16) und/oder stromaufwärts der Fördereinrichtung (10, 40, 150, 500) angeordnet ist,
- ein Verlustmittel-Rückström-Ventil (8), das vorzugsweise stromabwärts der Fördereinrichtung (10, 40, 150, 500) und/oder stromaufwärts der Verbindungseinrichtung (V) angeordnet ist,
- eine Durchflussmesseinrichtung (11b) zum Messen eines Volumenstroms.

12. Vorrichtung (100) nach einem der vorhergehenden Ansprüche, **dadurch gekennzeichnet, dass** die Vorrichtung (100) einen Spülmittelanschluss (A) zur Verbindung mit einem Spülmittel-Behälter (15) zur Aufnahme eines Spülmittels aufweist, um in einem Spülmodus zumindest abschnittsweise die Desinfektionsmittel-Leitung (L1) und/oder die Verlustmittel-Leitung (L2) zu spülen.

13. Vorrichtung (100) nach Anspruch 12, **dadurch gekennzeichnet, dass** der Spülmittelanschluss (A) über eine Spülmittel-Leitung (L4) mit der Desinfektionsmittel-Leitung (L1) in Verbindung bringbar ist, wobei vorzugsweise die Spülmittel-Leitung (L4) vor Mündung in die Desinfektionsmittel-Leitung (L1) ein Spülmittel-Ventil (6) umfasst.

14. Vorrichtung (100) nach einem der vorhergehenden Ansprüche, **dadurch gekennzeichnet, dass** die Vorrichtung (100)
- in einem Rückgewinnungs-Modus zum Rückgewinnen von durch den Desinfektionsprozess entstehendem Verlustmittel betreibbar ist, und/oder
- in einem Spülmodus zum zumindest abschnittsweisen Spülen der Desinfektionsmittel-Leitung (L1) und/oder der Verlustmittel-Leitung (L2) betreibbar ist.

15. Vorrichtung (100) nach einem der Ansprüche 5 bis 14, **dadurch gekennzeichnet, dass** die Fördereinrichtung (10, 40, 150, 500) im Rückgewinnungs-Modus zum Fördern des Verlustmittels aus dem Aufnahmebehälter (13) dient und/oder im Spülmodus zum Fördern des Spülmittels aus dem Spülmittel-Behälter (15) dient.

16. Vorrichtung (100) nach einem der Ansprüche 5 bis 15, **dadurch gekennzeichnet, dass** die Verlustmittel-Leitung (L2) an zwei Knotenpunkten (P1, P2) mit der Desinfektionsmittel-Leitung (L1) verbunden ist, wobei der erste Knotenpunkt (P1) zwischen der Fördereinrichtung (10, 40, 150, 500) und einem in der Desinfektionsmittel-Leitung (L1) stromabwärts der Fördereinrichtung (10, 40, 150, 500) angeordneten Glockenteller-Ventil (9) angeordnet ist und der zweite Knotenpunkt (P2) zwischen der Fördereinrichtung (10, 40, 150, 500) und einem in der Desinfektionsmittel-Leitung (L1) stromaufwärts der Fördereinrichtung (10, 40, 150, 500) angeordneten Desinfektionsmittel-Ventil (5) angeordnet ist.

17. Vorrichtung (100) nach einem der Ansprüche 5 bis 16, **dadurch gekennzeichnet, dass** die Spülmittel-Leitung (L4) an einem Knotenpunkt (P3) mit der Desinfektionsmittel-Leitung (L1) verbunden ist, wobei der Knotenpunkt (P3) zwischen der Fördereinrichtung (10, 40, 150, 500) und einem in der Desinfektionsmittel-Leitung (L1) stromaufwärts der Fördereinrichtung (10, 40, 150, 500) angeordneten Desinfektionsmittel-Ventil (5) angeordnet ist.

18. Vorrichtung (100) nach einem der vorhergehenden Ansprüche, **dadurch gekennzeichnet, dass** die Vorrichtung (100) einen Multi-Behälter (50) zur Aufnahme des Desinfektionsmittels für den Desinfektionsprozess aufweist und der Multi-Behälter (50) zusätzlich zur Aufnahme von Spülmittel für einen Spülmodus dient, so dass der Multi-Behälter (50) für den Desinfektionsprozess mit dem Desinfektionsmittel und für den Spülmodus mit dem Spülmittel befüllbar und betreibbar ist.

19. Vorrichtung (100) nach Anspruch 18, **dadurch gekennzeichnet, dass** der Multi-Behälter (50) in die Desinfektionsmittel-Leitung (L1) und/oder die Spülmittel-Leitung (L4) integrierbar ist.

20. Vorrichtung (100) nach Anspruch 18 oder 19, **dadurch gekennzeichnet, dass** der Multi-Behälter (50), vorzugsweise über das Desinfektionsmittel-Ventil 5), mit der Verbindungseinrichtung (V) in Verbindung bringbar ist und/oder, vorzugsweise über das Spülmittel-Ventil (6), mit dem Spülmittelanschluss (A) in Verbindung bringbar ist.

21. Vorrichtung (100) nach einem der vorhergehenden Ansprüche, **dadurch gekennzeichnet, dass** der Glockenteller (1) mit einer hohlen Antriebswelle (S) zum Antreiben des Glockentellers (1) verbunden ist und vorzugsweise eine Zuleitung in der hohle Antriebswelle (S) Teil der Desinfektionsmittel-Leitung (L1) ist, die Zuführung des Desinfektionsmittels zum Glockenteller (1) durch die hohle Antriebswelle (S) hindurch erfolgt und/oder eine Desinfektionsmittel-Austrittsdüse (D) umfasst und die Desinfektionsmittel-Austrittsdüse (D) in einer Basis (B) des Glockentellers (1) aufgenommen ist.

22. Vorrichtung (100) nach einem der vorhergehenden Ansprüche, **dadurch gekennzeichnet, dass** der Glockenteller (1) mit einer rotierbaren Platte (P) zusammenwirkt, über die das Desinfektionsmittel zum Glockenteller (1) verteilt wird.

23. Vorrichtung (100) nach einem der vorhergehenden Ansprüche, **dadurch gekennzeichnet, dass** der Weg des Desinfektionsmittels zum Glockenteller (1) zumindest eines von Folgendem umfasst:
- ein Desinfektionsmittel-Ventil (5) stromaufwärts der Fördereinrichtung (10, 40, 150, 500),
- ein Glockenteller-Ventil (9) stromabwärts der Fördereinrichtung (10, 40, 150, 500),
- die Fördereinrichtung (10, 40, 150, 500),
- den Elektromotor (2) oder die Luftturbine,
- die hohle Antriebswelle (S) mit Desinfektionsmittel-Austrittsdüse (D),
- zumindest eine Durchflussmesseinrichtung (11a, 11b) zum Messen eines Volumenstroms,
- die rotierbare Platte (P).

24. Vorrichtung (100) nach einem der Ansprüche 5 bis 23, **dadurch gekennzeichnet, dass** die Fördereinrichtung (10, 40, 150,500) elektrisch, pneumatisch oder hydraulisch angetrieben ist und zumindest eine Pumpe (10) und/oder zumindest ein Kolbensystem (40, 150, 500) umfasst.

25. Vorrichtung (100) nach einem der Ansprüche 12 bis 24, **dadurch gekennzeichnet, dass** der Weg des Spülmittels im Spülmodus zumindest eines von Folgendem umfasst:
- das Spülmittel-Ventil (6),
- die Fördereinrichtung (10, 40, 150, 500),
- einen Teilabschnitt der Desinfektionsmittel-Leitung (L1),
- die hohle Antriebswelle (S),
- einen Teilabschnitt der Verlustmittel-Leitung (L2),
- zumindest eine Durchflussmesseinrichtung (11a, 11b),
- das in der Desinfektionsmittel-Leitung (L1) stromabwärts der Fördereinrichtung (10, 40, 150, 500) angeordnete Glockenteller-Ventil (9),
- den Elektromotor (2) oder die Luftturbine,
- den Glockenteller (1),
- die rotierbare Platte (P),
- das in der Verlustmittel-Leitung (L2) stromabwärts der Fördereinrichtung (10, 40, 150, 500) angeordnete Verlustmittel-Rückström-Ventil (8),
- die Verbindungseinrichtung (V).

26. Vorrichtung (100) nach Anspruch 24 oder 25, **dadurch gekennzeichnet, dass** die Pumpe (10) - das Desinfektionsmittel aus dem Desinfektionsmittel-Behälter (4),
- das Spülmittel aus dem Spülmittel-Behälter (15) und/oder
- das Verlustmittel aus dem Aufnahmebehälter (13) fördert.

27. Vorrichtung (100) nach einem der Ansprüche 24 bis 26, **dadurch gekennzeichnet, dass** das Kolbensystem (40, 150, 500) zumindest eines von Folgendem umfasst:
- ein Desinfektionsmittel-Kolbensystem (40), wobei vorzugsweise der Desinfektionsmittel-Behälter (4) in das Desinfektionsmittel-Kolbensystem (40) integrierbar ist, und/oder
- ein Spülmittel-Kolbensystem (150), wobei vorzugsweise der Spülmittel-Behälter (15) in das Spülmittel-Kolbensystem (150) integrierbar ist,
- ein Desinfektionsmittel-Spülmittel-Kolbensystem (500), wobei vorzugsweise der Multi-Behälter (50) in das Desinfektionsmittel-Spülmittel-Kolbensystem (500) integrierbar ist.

28. Vorrichtung (100) nach einem der Ansprüche 3 bis 27, **dadurch gekennzeichnet, dass** die Vorrichtung (100) zumindest ein stromaufwärts des Glockentellers (1) angeordnetes Gebläse (3) zum Transportieren und Verteilen von mittels des Glockentellers (1) zerstäubtem Desinfektionsmittel aufweist und/oder zumindest ein stromabwärts des Glockentellers (1) angeordnetes Gebläse (21) zum Transportieren und Verteilen von mittels des Glockentellers (1) zerstäubtem Desinfektionsmittel aufweist.

29. Vorrichtung (100) nach einem der Ansprüche 3 bis 28, **dadurch gekennzeichnet, dass** das Gebläse (3) stromaufwärts des Glockentellers (1) koaxial zum Glockenteller (1) angeordnet ist und/oder das Gebläse (21) stromabwärts des Glockentellers (1) nicht-koaxial zum Glockenteller (1) angeordnet ist und zum Verteilen des zerstäubten Desinfektionsmittels zur Seite hin dient.

30. Vorrichtung (100) nach einem der vorhergehenden Ansprüche, **dadurch gekennzeichnet, dass** die Vorrichtung (100) eine Feuchtigkeitsmesseinrichtung (19) mit Regelwirkung auf einen Desinfizierungsprozess der Vorrichtung (100) aufweist, wobei die Feuchtigkeitsmesseinrichtung (19) zum Messen einer Feuchtigkeit in dem Raum dient, und/oder dass die Vorrichtung (100) eine Temperaturmesseinrichtung (20) mit Regelwirkung auf einen Desinfizierungsprozess der Vorrichtung (100) aufweist, wobei die Temperaturmesseinrichtung (20) zum Messen einer Temperatur in dem zumindest einen Raum dient.

31. Vorrichtung (100) nach einem der Ansprüche 1 bis 30, **dadurch gekennzeichnet, dass** die Vorrichtung (100) zumindest eine Füllstandsmesseinrichtung (17a, 17b) aufweist zum Messen eines in dem Desinfektionsmittel-Behälter (4) vorliegenden Füllstands, zum Messen eines in dem Aufnahmebehälter (13) vorliegenden Füllstands aufweist und/oder zum Messen eines in dem Spülmittel-Behälter (15) vorliegenden Füllstands aufweist.

32. Vorrichtung (100) nach einem der vorhergehenden Ansprüche, **dadurch gekennzeichnet, dass** der Desinfektionsmittel-Behälter (4), der Spülmittel-Behälter (15) und/oder der Multi-Behälter (50) als wechselbarer Einweg-Behälter ausgeführt ist oder als fest in die Vorrichtung (100) integrierter Behälter ausgeführt ist.

33. Vorrichtung (100) nach einem der vorhergehenden Ansprüche, **dadurch gekennzeichnet, dass** der Glockenteller (1)
- im Betrieb eine Drehzahl von mindestens 30.000 Umdrehungen pro Minute aufweist,
- eine Zerstäubungsleistung von über 50 Milliliter pro Minute aufweist,
- ein umgewälztes Luftvolumen von über 0,3 Kubikmeter pro Minute aufweist, und/oder
- eine ringförmige Glockentellerabspritzkante (E) zum Abspritzen des Desinfektionsmittels aufweist und die Glockentellerabspritzkante (E) vorzugsweise einen Durchmesser zwischen 40mm und 120mm aufweist.

34. Vorrichtung (100) nach einem der vorhergehenden Ansprüche, **dadurch gekennzeichnet, dass** eine Innenfläche (1a) des Glockentellers (1)
- einen Innenwinkel (alpha) zwischen 5° und 45° aufweist, und/oder
- einen Randbereich aufweist, wobei der Randbereich an die Glockentellerabspritzkante (E) anschließt und eine Breite (X) von zwischen 0,05 mm und 10 mm aufweist und nur der Randbereich mit einer Oberflächenstruktur für das Desinfektionsmittel versehen ist.

35. Vorrichtung (100) nach einem der vorhergehenden Ansprüche, **dadurch gekennzeichnet, dass** die Vorrichtung (100) zumindest eine Raumvermessungseinrichtung zum Vermessen des Raums aufweist, wobei in Abhängigkeit von Messdaten der Raumvermessungseinrichtung ein Desinfizierungsprozess der Vorrichtung (100) erfolgt oder eine Einleitung eines Desinfizierungsprozesses der Vorrichtung (100) unterbunden wird.

36. Vorrichtung (100) nach einem der vorhergehenden Ansprüche, **dadurch gekennzeichnet, dass** die Vorrichtung (100) einen Gasleitring (R) mit zumindest einer Leckageöffnung (N) zur Ausgabe eines Gases aufweist und der Gasleitring (R) zur Leitung des Gases an einer Außenfläche des Glockentellers (1) dient, wobei das Gas auf das vom Glockenteller (1) abgegebene Desinfektionsmittel trifft, um das Desinfektionsmittel zu formen und/oder dessen Tröpfchengröße zu reduzieren.

37. Vorrichtung (100) nach Anspruch 36, **dadurch gekennzeichnet, dass** der Gasleitring (R) eine Vielzahl an ringförmig angeordneten Leckageöffnungen (N) aufweist und/oder eine Basis (B) des Glockentellers (1) und einen Teil einer Außenfläche (1b) des Glockentellers (1) ummantelt.

38. Vorrichtung (100) nach einem der vorhergehenden Ansprüche, **dadurch gekennzeichnet, dass** die Vorrichtung (100) zumindest eine Erkennungseinrichtung umfasst, zum Erkennen des Desinfektionsmittels, zum Erkennen des Spülmittels und/oder zum Erkennen einer Drehzahl des Glockentellers (1).

39. Vorrichtung (100) nach einem der vorhergehenden Ansprüche, **dadurch gekennzeichnet, dass** die Vorrichtung (100) zum Zerstäuben des Desinfektionsmittels in Tröpfchen mit einer Größe von zwischen 1µm und 50µm, vorzugsweise zwischen 3 µm und 10 µm ausgebildet ist.

40. Vorrichtung (100) nach einem der vorhergehenden Ansprüche, **dadurch gekennzeichnet, dass** die Vorrichtung (100) eine portable Steuereinheit aufweist, mittels der die Vorrichtung (100) über eine Kabelverbindung oder kabellos steuerbar ist.

## Claims

1. Apparatus (100) for disinfecting at least one room, in particular for one or more persons, , with
a) an atomiser, which includes a rotatable bell cup (1) for atomising a disinfectant into the room,
b) a disinfectant line (L1) which can be connected to the bell cup (1) in order to supply the disinfectant to the bell cup (1),
c) a connecting device (V) for connecting to a disinfectant-container (4) for holding the disinfectant, wherein the connecting device (V) can be connected with the bell cup (1) via the disinfectant line (L1),
d) wherein the apparatus (100) comprises a receiving container (13) for receiving liquid lost during the disinfection process,
e) wherein the apparatus (100) comprises a lost-liquid line (L2)
f) wherein the receiving container (13) can be emptied via the lost-liquid line (L2),
**characterized in**
g) **that** the receiving container (13) can be connected to the connecting device (V), and
h) **that** the lost liquid from the receiving container (13) can be led via the connecting device (V) to the disinfectant-container (4), which was previously emptied in the disinfection process, for disposal.

2. Apparatus (100) according to claim 1, **characterised in that** the apparatus (100) includes an electric motor (2) and/or an air turbine for driving the bell cup (1).

3. Apparatus (100) according to claim 1 or 2, **characterised in that** the apparatus (100) includes at least one blower (3, 21), wherein the blower (3, 21) serves to convey and distribute disinfectant atomised by means of the bell cup (1).

4. Apparatus (100) according to one of the preceding claims, **characterised in that** the bell cup (1), the electric motor (2), the air turbine and/or the blower (3) are housed at least partially in an atomiser housing (Z).

5. Apparatus (100) according to one of the preceding claims, **characterised in that** the apparatus (100) includes at least one delivery device (10, 40, 150, 500) for conveying and/or dosing the disinfectant for the disinfection process.

6. Apparatus (100) according to one of the preceding claims, **characterised in that** the apparatus (100) includes at least one flow measuring device (11a, 11b) for measuring a volume flow, wherein preferably
- a flow measuring device (11a) is disposed between the bell cup (1) and a bell cup valve (9) disposed downstream of the delivery device (10, 40, 150, 500), and/or
- a flow measuring device (11b) is disposed between the delivery device (10, 40, 150, 500) and a bell cup valve (9) disposed downstream of the delivery device (10, 40, 150, 500).

7. Apparatus (100) according to claim 10, **characterised in that** the receiving container (13) serves to receive liquid deposited from the disinfectant on or in the atomiser housing (Z), wherein preferably the deposited liquid can be led to the receiving container (13) via a funnel (12).

8. Apparatus (100) according to one of the preceding claims, **characterised in that** the apparatus (100) includes at least one air dehumidifier (14) for dehumidifying air in the room, preferably with a total dehumidification capacity of over 600 or over 700 cubic metres per hour.

9. Apparatus (100) according to claim 8, **characterised in that** the at least one air dehumidifier (14) can be connected to the receiving container (13) in order to supply moisture extracted from the air to the receiving container (13).

10. Apparatus (100) according to one of the preceding claims, **characterised in that** the delivery device (10, 40, 150, 500) can be connected to the receiving container (13) via a line (L3) in order to supply liquid to the receiving container (13) via the line (L3) when excess pressure is present at a pressure relief valve (18).

11. Apparatus (100) according to claim 1, **characterised in that** the way of the lost liquid on its way from the receiving container (13) to the connecting device (V) comprises at least one of the following:
- a filter device (16) for filtering the lost liquid,
- the delivery device (10, 40, 150, 500),
- a receiving container valve (7), which is preferably disposed downstream of the filter device (16) and/or upstream of the delivery device (10, 40, 150, 500),
- a lost-liquid return valve (8), which is preferably disposed downstream of the delivery device (10, 40, 150, 500) and/or upstream of the connecting device (V),
- a flow measuring device (11b) for measuring a volume flow.

12. Apparatus (100) according to one of the preceding claims, **characterised in that** the apparatus (100) has a rinsing agent connection (A) for connecting with a rinsing agent container (15) for receiving a rinsing agent in order to rinse, while in a rinsing mode, at least sections of the disinfectant line (L1) and/or the lost-liquid line (L2).

13. Apparatus (100) according to claim 12, **characterised in that** the rinsing agent connection (A) can be connected to the disinfectant line (L1) via a rinsing agent line (L4), the rinsing agent line (L4) preferably including a rinsing agent valve (6) before leading into the disinfectant line (L1).

14. Apparatus (100) according to one of the preceding claims, **characterised in that** the apparatus (100)
- is operable in a recovery mode for recovering lost liquid resulting from the disinfection process, and/or
- is operable in a rinsing mode for rinsing at least sections of the disinfectant line (L1) and/or the lost-liquid line (L2).

15. Apparatus (100) according to one of claims 5 to 14, **characterised in that** the delivery device (10, 40, 150, 500) serves in recovery mode to convey lost liquid from the receiving container (13) and/or serves in rinsing mode to convey rinsing agent from the rinsing agent container (15).

16. Apparatus (100) according to one of claims 5 to 15, **characterised in that** the lost-liquid line (L2) is connected to the disinfectant line (L1) at two junction points (P1, P2), the first junction point (P1) being disposed between the delivery device (10, 40, 150, 500) and a bell cup valve (9) disposed in the disinfectant line (L1) downstream of the delivery device (10, 40, 150, 500), and the second junction point (P2) being disposed between the delivery device (10, 40, 150, 500) and a disinfectant valve (5) disposed in the disinfectant line (L1) upstream of the delivery device (10, 40, 150, 500).

17. Apparatus (100) according to one of claims 5 to 16, **characterised in that** the rinsing agent line (L4) is connected to the disinfectant line (L1) at a junction point (P3), the junction point (P3) being disposed between the delivery device (10, 40, 150, 500) and a disinfectant valve (5) disposed in the disinfectant line (L1) upstream of the delivery device (10, 40, 150, 500).

18. Apparatus (100) according to one of the preceding claims, **characterised in that** the apparatus (100) has a multi-functional container (50) for holding disinfectant for the disinfection process and the multi-functional container (50) in addition serves to hold rinsing agent for a rinsing mode, so that the multi-functional container (50) can be filled and operated with disinfectant for the disinfection process and with rinsing agent while in a rinsing mode.

19. Apparatus (100) according to claim 18, **characterised in that** the multi-functional container (50) is integrable into the disinfectant line (L1) and/or the rinsing agent line (L4).

20. Apparatus (100) according to claim 18 or 19, **characterised in that** the multi-functional container (50) is connectable with the connecting device (V), preferably via the disinfectant valve (5), and/or with the rinsing agent connection (A), preferably via the rinsing agent valve (6).

21. Apparatus (100) according to one of the preceding claims, **characterised in that** the bell cup (1) is connected to a hollow drive shaft (S) for driving the bell cup (1) and preferably a supply line in the hollow drive shaft (S) is part of the disinfectant line (L1), the disinfectant supply to the bell cup (1) through the hollow drive shaft (S) and/or includes a disinfectant outlet nozzle (D), and the disinfectant outlet nozzle (D) is housed in a base (B) of the bell cup (1).

22. Apparatus (100) according to one of the preceding claims, **characterised in that** the bell cup (1) works in conjunction with a rotatable cup (P) via which the disinfectant is distributed to the bell cup (1).

23. Apparatus (100) according to one of the preceding claims, **characterised in that** the way of the disinfectant to the bell cup (1) comprises at least one of the following:
- a disinfectant valve (5) upstream of the delivery device (10, 40, 150, 500),
- a bell cup valve (9) downstream of the delivery device (10, 40, 150, 500),
- the delivery device (10, 40, 150, 500),
- the electric motor (2) or the air turbine,
- the hollow drive shaft (S) with disinfectant outlet nozzle (D)
- at least one flow measuring device (11a, 11b) for measuring a volume flow,
- the rotatable cup (P).

24. Apparatus (100) according to one of claims 5 to 23, **characterised in that** the delivery device (10, 40, 150, 500) is driven electrically, pneumatically or hydraulically and includes at least one pump (10) and/or at least one piston system (40, 150, 500).

25. Apparatus (100) according to one of claims 12 to 24, **characterised in that** the rinsing agent passes at least one of the following in rinsing mode:
- the rinsing agent valve (6),
- the delivery device (10, 40, 150, 500),
- a section of the disinfectant line (L1),
- the hollow drive shaft (S),
- a section of the lost-liquid line (L2),
- at least one flow measuring device (11a, 11b),
- the bell cup valve (9) disposed in the disinfectant line (L1) downstream of the delivery device (10, 40, 150, 500),
- the electric motor (2) or the air turbine,
- the bell cup (1),
- the rotatable cup (P),
- the lost-liquid recovery valve (8) disposed in the lost-liquid line (L2) downstream of the delivery device (10, 40, 150, 500),
- the connecting device (V).

26. Apparatus (100) according to claim 24 or 25, **characterised in that** the pump (10) serves to convey at least one of the following:
- disinfectant from the disinfectant container (4),
- rinsing agent from the rinsing agent container (15),
- lost liquid from the receiving container (13).

27. Apparatus (100) according to one of claims 24 to 26, **characterised in that** the piston system (40, 150, 500) includes at least one of the following:
- a disinfectant piston system (40), wherein preferably the disinfectant container (4) is integrable into the disinfectant piston system (40), and/or
- a rinsing agent piston system (150), wherein preferably the rinsing agent container (15) is integrable into the rinsing agent piston system (150),
- a disinfectant-rinsing agent piston system (500), wherein preferably the multi-functional container (50) is integrable into the disinfectant-rinsing agent piston system (500).

28. Apparatus (100) according to one of claims 3 to 27, **characterised in that** the apparatus (100) includes at least one blower (3) disposed upstream of the bell cup (1) for transporting and distributing disinfectant atomised by means of the bell cup (1) and/or at least one blower (21) disposed downstream of the bell cup (1) for transporting and distributing disinfectant atomised by means of the bell cup (1).

29. Apparatus (100) according to one of claims 3 to 28, **characterised in that** the blower (3) is disposed upstream of the bell cup (1) coaxially with the bell cup (1) and/or the blower (21) is disposed downstream of the bell cup (1) non-coaxially with the bell cup (1) and serves to distribute the atomised disinfectant sideways.

30. Apparatus (100) according to one of the preceding claims, **characterised in that** the apparatus (100) includes a humidity measuring device (19) with regulating effect on the disinfection process of the apparatus (100), the humidity measuring device (19) serving to measure humidity in the room, and/or
that the apparatus (100) includes a temperature measuring device (20) with regulating effect on the disinfection process of the apparatus (100), the temperature measuring device (20) serving to measure temperature in the at least one room.

31. Apparatus (100) according to one of claims 1 to 30, **characterised in that** the apparatus (100) includes at least one level measuring device (17a, 17b) for measuring a fill level in the disinfectant container (4), for measuring a fill level in the receiving container (13) and/or for measuring a fill level in the rinsing agent container (15).

32. Apparatus (100) according to one of the preceding claims, **characterised in that** the disinfectant container (4), the rinsing agent container (15) and/or the multi-functional container (50) is designed as a replaceable single-use container or as a container permanently integrated into the apparatus (100).

33. Apparatus (100) according to one of the preceding claims, **characterised in that** the bell cup (1)
- has a rotation speed of at least 30,000 revolutions per minute in operation,
- has an atomising capacity of more than 50 millilitres per minute,
- has a circulating air volume of more than 0.3 cubic metres per minute, and/or
- has an annular bell cup spray edge (E) for spraying disinfectant and the bell cup spray edge (E) preferably has a diameter between 40 mm and 120 mm.

34. Apparatus (100) according to one of the preceding claims, **characterised in that** an inner face (1a) of the bell cup (1)
- has an internal angle alpha between 5° and 45°, and/or
- has a peripheral zone whereby the peripheral zone adjoins the bell cup spray edge E and has a width (X) of between 0.05 mm and 10 mm and only the peripheral zone is provided with a surface structure for the disinfectant.

35. Apparatus (100) according to one of the preceding claims, **characterised in that** the apparatus (100) has at least one room measuring device for measuring the room, the apparatus (100) carrying out a disinfection process or the apparatus (100) being prevented from starting a disinfection process depending on measurement data from the room measuring device.

36. Apparatus (100) according to one of the preceding claims, **characterised in that** the apparatus (100) includes a gas directing ring (R) having at least one leakage opening (N) for discharging a gas, and the gas directing ring (R) serves to direct the gas on an outer face of the bell cup (1), the gas meeting the disinfectant discharged from the bell cup (1) to shape the disinfectant into the required form and/or reduce its droplet size.

37. Apparatus (100) according to claim 36, **characterised in that** the gas directing ring (R) has a plurality of annularly disposed leakage openings (N) and/or encases a base (B) of the bell cup (1) and a part of an outer face (1b) of the bell cup (1).

38. Apparatus (100) according to one of the preceding claims, **characterised in that** the apparatus (100) includes at least one detection device, for detecting the disinfectant, for detecting the rinsing agent and/or for detecting a rotation speed of the bell cup (1).

39. Apparatus (100) according to one of the preceding claims, **characterised in that** the apparatus (100) is adapted to atomise the disinfectant into droplets having a size of between 1µm and 50µm, preferably between 3µm and 10µm.

40. Apparatus (100) according to one of the preceding claims, **characterised in that** the apparatus (100) includes a portable control unit which allows the apparatus (100) to be controlled via a wired or wireless connection.

## Revendications

1. Dispositif (100) pour désinfecter au moins une pièce, en particulier pour une ou plusieurs personnes, avec
a) un pulvérisateur comprenant un plateau à cloche rotatif (1) pour pulvériser un agent désinfectant dans l'espace,
b) une conduite d'agent désinfectant (L1) qui peut être raccordée au plateau à cloche (1) afin d'amener l'agent désinfectant au plateau à cloche (1), et
c) un dispositif de raccordement (V) destiné à être raccordé à un réservoir d'agent désinfectant (4) qui doit recevoir l'agent désinfectant, le dispositif de raccordement (V) pouvant être raccordé au plateau à cloche (1) par l'intermédiaire de la conduite d'agent désinfectant (L1),
d) le dispositif (100) présentant un récipient de réception (13) destiné à recevoir le fluide de perte généré par le processus de désinfection,
e) le dispositif (100) comprenant une conduite de fluide de perte (L2),
f) le récipient de réception (13) pouvant être vidé via la conduite de fluide de perte (L2), **caractérisé en ce que**
g) le récipient de réception (13) peut être raccordé au dispositif de raccordement (V), et
h) le fluide de perte provenant du récipient de réception (13) peut être amené par l'intermédiaire du dispositif de raccordement (V) au réservoir d'agent désinfectant (4) préalablement vidé au cours du processus de désinfection, en vue de l'élimination.

2. Dispositif (100) selon la revendication 1, **caractérisé en ce que** le dispositif (100) présente un moteur électrique (2) et/ou une turbine à air pour entraîner le plateau à cloche (1).

3. Dispositif (100) selon la revendication 1 ou 2, **caractérisé en ce que** le dispositif (100) présente au moins une soufflante (3, 21), la soufflante (3, 21) servant à transporter et à répartir l'agent désinfectant pulvérisé au moyen du plateau à cloche (1).

4. Dispositif (100) selon l'une des revendications précédentes, **caractérisé en ce que** le plateau à cloche (1), le moteur électrique (2), la turbine à air et/ou la soufflante (3) sont logés, au moins par sections, dans un boîtier de pulvérisateur (Z).

5. Dispositif (100) selon l'une des revendications précédentes, **caractérisé en ce que** le dispositif (100) présente au moins un dispositif de transport (10, 40, 150, 500) pour transporter et/ou doser l'agent désinfectant pour le processus de désinfection.

6. Dispositif (100) selon l'une des revendications précédentes, **caractérisé en ce que** le dispositif (100) présente au moins un dispositif de mesure de débit (11a, 11b) pour mesurer un débit volumétrique, de préférence
- un dispositif de mesure de débit (11a) étant agencé entre le plateau à cloche (1) et une vanne de plateau à cloche (9) agencée en aval du dispositif de transport (10, 40, 150, 500), et/ou
- un dispositif de mesure de débit (11b) étant agencé entre le dispositif de transport (10, 40, 150, 500) et une vanne de plateau à cloche (9) agencée en aval du dispositif de transport (10, 40, 150, 500).

7. Dispositif (100) selon la revendication 1, **caractérisé en ce que** le récipient de réception (13) sert à recevoir le fluide séparé de l'agent désinfectant sur ou dans le boîtier de pulvérisation (Z), le fluide séparé pouvant de préférence être amené au récipient de réception (13) par un entonnoir (12).

8. Dispositif (100) selon l'une des revendications précédentes, **caractérisé en ce que** le dispositif (100) présente au moins un déshumidificateur (14) pour déshumidifier l'air dans la pièce, avec de préférence une capacité de déshumidification totale supérieure à 600 ou à 700 mètres cubes par heure.

9. Dispositif (100) selon la revendication 8, **caractérisé en ce que** ledit au moins un déshumidificateur d'air (14) peut être raccordé au récipient de réception (13) afin d'amener l'humidité extraite de l'air au récipient de réception (13).

10. Dispositif (100) selon l'une des revendications précédentes, **caractérisé en ce que** le dispositif de transport (10, 40, 150, 500) peut être raccordé au récipient de réception (13) par l'intermédiaire d'une conduite (L3), afin d'amener du fluide au récipient de réception (13) par l'intermédiaire de la conduite (L3) lorsqu'une surpression est présente au niveau d'une soupape de surpression (18).

11. Dispositif (100) selon la revendication 1, **caractérisé en ce que** le trajet du fluide de perte depuis le récipient de réception (13) jusqu'au dispositif de raccordement (V) comprend au moins l'un des éléments suivants :
- un dispositif de filtrage (16) pour filtrer le fluide de perte,
- le dispositif de transport (10, 40, 150, 500),
- une vanne de réservoir de réception (7) agencée de préférence en aval du dispositif de filtrage (16) et/ou en amont du dispositif de transport (10, 40, 150, 500), une vanne de retour de fluide de perte (8) agencée de préférence en aval du dispositif de transport (10, 40, 150, 500) et/ou en amont du dispositif de raccordement (V),
- un dispositif de mesure de débit (11b) pour mesurer un débit volumétrique.

12. Dispositif (100) selon l'une des revendications précédentes, **caractérisé en ce que** le dispositif (100) présente un raccord de produit de rinçage (A) destiné à être relié à un réservoir de produit de rinçage (15) destiné à recevoir un produit de rinçage afin de rincer, au moins par tronçons, la conduite d'agent désinfectant (L1) et/ou la conduite de fluide de perte (L2) dans un mode de rinçage.

13. Dispositif (100) selon la revendication 12, **caractérisé en ce que** le raccord de produit de rinçage (A) peut être raccordé à la conduite d'agent désinfectant (L1) par l'intermédiaire d'une conduite de produit de rinçage (L4), la conduite de produit de rinçage (L4) comprenant de préférence une vanne de produit de rinçage (6) avant de déboucher dans la conduite d'agent désinfectant (L1).

14. Dispositif (100) selon l'une quelconque des revendications précédentes, **caractérisé en ce que** le dispositif (100)
- peut fonctionner dans un mode de récupération pour récupérer le fluide de perte généré par le processus de désinfection, et/ou
- peut fonctionner dans un mode de rinçage pour rincer au moins par sections la conduite d'agent désinfectant (L1) et/ou la conduite de fluide de perte (L2).

15. Dispositif (100) selon l'une des revendications 5 à 14, **caractérisé en ce que** le dispositif de transport (10, 40, 150, 500) sert, en mode de récupération, à transporter le fluide de perte hors du récipient de réception (13) et/ou, en mode de rinçage, à transporter le produit de rinçage hors du récipient de produit de rinçage (15).

16. Dispositif (100) selon l'une quelconque des revendications 5 à 15, **caractérisé en ce que** la conduite de fluide de perte (L2) est reliée à la conduite d'agent désinfectant (L1) en deux points nodaux (P1, P2), le premier point nodal (P1) étant situé entre le dispositif de transport (10, 40, 150, 500) et une vanne de plateau à cloche (9) agencée dans la conduite d'agent désinfectant (L1) en aval du dispositif de transport (10, 40, 150, 500), et le deuxième point nodal (P2) est agencé entre le dispositif de transport (10, 40, 150, 500) et une vanne d'agent désinfectant (5) agencée dans la conduite d'agent désinfectant (L1) en amont du dispositif de transport (10, 40, 150, 500).

17. Dispositif (100) selon l'une des revendications 5 à 16, **caractérisé en ce que** la conduite de produit de rinçage (L4) est raccordée à la conduite d'agent désinfectant (L1) au niveau d'un point de jonction (P3), le point de jonction (P3) étant situé entre le dispositif de transport (10, 40, 150, 500) et une vanne d'agent désinfectant (5) agencée dans la conduite d'agent désinfectant (L1) en amont du dispositif de transport (10, 40, 150, 500).

18. Dispositif (100) selon l'une des revendications précédentes, **caractérisé en ce que** le dispositif (100) présente un multi-récipient (50) destiné à recevoir l'agent désinfectant pour le processus de désinfection et le multi-récipient (50) sert en outre à recevoir le produit de rinçage pour un mode de rinçage, de sorte que le multi-récipient (50) peut être rempli et utilisé avec l'agent désinfectant pour le processus de désinfection et avec le produit de rinçage pour le mode de rinçage.

19. Dispositif (100) selon la revendication 18, **caractérisé en ce que** le multi-récipient (50) peut être intégré dans la conduite d'agent désinfectant (L1) et/ou dans la conduite de produit de rinçage (L4).

20. Dispositif (100) selon la revendication 18 ou 19, **caractérisé en ce que** le multi-récipient (50) peut être raccordé, de préférence par l'intermédiaire de la vanne d'agent désinfectant (5), au dispositif de raccordement (V) et/ou peut être raccordé, de préférence par l'intermédiaire de la vanne de produit de rinçage (6), au raccord de produit de rinçage (A).

21. Dispositif (100) selon l'une des revendications précédentes, **caractérisé en ce que** le plateau à cloche (1) est raccordé à un arbre d'entraînement creux (S) pour entraîner le plateau à cloche (1) et, de préférence, une conduite d'alimentation dans l'arbre d'entraînement creux (S) fait partie de la conduite d'agent désinfectant (L1), l'acheminement de l'agent désinfectant au plateau à cloche (1) s'effectuant à travers l'arbre d'entraînement creux (S) et/ou comprenant une buse de sortie d'agent désinfectant (D) et la buse de sortie d'agent désinfectant (D) étant logée dans une base (B) du plateau à cloche (1).

22. Dispositif (100) selon l'une des revendications précédentes, **caractérisé en ce que** le plateau à cloche (1) coopère avec un plateau rotatif (P) par lequel le produit désinfectant est distribué vers le plateau à cloche (1).

23. Dispositif (100) selon l'une des revendications précédentes, **caractérisé en ce que** le trajet de l'agent désinfectant vers le plateau à cloche (1) comprend au moins l'un des éléments suivants :
- une vanne d'agent désinfectant (5) en amont du dispositif de transport (10, 40, 150, 500),
- une vanne de plateau à cloche (9) en aval du dispositif de transport (10, 40, 150, 500),
- le dispositif de transport (10, 40, 150, 500),
- le moteur électrique (2) ou la turbine à air,
- l'arbre d'entraînement creux (S) avec buse de sortie d'agent désinfectant (D),
- au moins un dispositif de mesure de débit (11a, 11b) pour mesurer un débit volumétrique,
- la plaque tournante (P).

24. Dispositif (100) selon l'une des revendications 5 à 23, **caractérisé en ce que** le dispositif de transport (10, 40, 150, 500) est entraîné électriquement, pneumatiquement ou hydrauliquement et comprend au moins une pompe (10) et/ou au moins un système de piston (40, 150, 500).

25. Dispositif (100) selon l'une quelconque des revendications 12 à 24, **caractérisé en ce que** le trajet du produit de rinçage en mode de rinçage comprend au moins l'un des éléments suivants :
- la vanne de produit de rinçage (6),
- le dispositif de transport (10, 40, 150, 500),
- une section de la conduite d'agent désinfectant (L1),
- l'arbre d'entraînement creux (S),
- une section de la conduite de fluide de perte (L2),
- au moins un dispositif de mesure de débit (11a, 11b),
- la vanne de plateau à cloche (9) agencée dans la conduite d'agent désinfectant (L1) en aval du dispositif de transport (10, 40, 150, 500)
- le moteur électrique (2) ou la turbine à air,
- le plateau à cloche (1),
- la plaque tournante (P),
- la vanne de retour de fluide de perte (8) agencée dans la conduite de fluide de perte (L2) en aval du dispositif de transport (10, 40, 150, 500),
- le dispositif de raccordement (V).

26. Dispositif (100) selon la revendication 24 ou 25, **caractérisé en ce que** la pompe (10)
- évacue l'agent désinfectant du réservoir d'agent désinfectant (4),
- évacue le produit de rinçage du réservoir de produit de rinçage (15) et/ou
- le fluide de perte du récipient de réception (13).

27. Dispositif (100) selon l'une quelconque des revendications 24 à 26, **caractérisé en ce que** le système de piston (40, 150, 500) comprend au moins l'un des éléments suivants :
- un système de piston d'agent désinfectant (40), le réservoir d'agent désinfectant (4) pouvant de préférence être intégré dans le système de piston d'agent désinfectant (40), et/ou
- un système de piston de produit de rinçage (150), le réservoir de produit de rinçage (15) pouvant de préférence être intégré dans le système de piston de produit de rinçage (150),
- un système de piston de produit de rinçage désinfectant (500) dans lequel, de préférence, le multi-récipient (50) peut être intégré dans le système de piston de produit de rinçage désinfectant (500).

28. Dispositif (100) selon l'une des revendications 3 à 27, **caractérisé en ce que** le dispositif (100) présente au moins une soufflante (3) agencée en amont du plateau à cloche (1) pour transporter et distribuer du produit désinfectant pulvérisé au moyen du plateau à cloche (1) et/ou présente au moins une soufflante (21) agencée en aval du plateau à cloche (1) pour transporter et distribuer du produit désinfectant pulvérisé au moyen du plateau à cloche (1).

29. Dispositif (100) selon l'une des revendications 3 à 28, **caractérisé en ce que** la soufflante (3) est agencée en amont du plateau à cloche (1), coaxialement au plateau à cloche (1), et/ou la soufflante (21) est agencée en aval du plateau à cloche (1), non coaxialisent au plateau à cloche (1), et sert à répartir l'agent désinfectant pulvérisé vers le côté.

30. Dispositif (100) selon l'une quelconque des revendications précédentes, **caractérisé en ce que** le dispositif (100) présente un dispositif de mesure d'humidité (19) ayant une action de régulation sur un processus de désinfection du dispositif (100), le dispositif de mesure d'humidité (19) servant à mesurer une humidité dans la pièce, et/ou **en ce que** le dispositif (100) présente un dispositif de mesure de température (20) ayant une action de régulation sur un processus de désinfection du dispositif (100), le dispositif de mesure de température (20) servant à mesurer une température dans ladite au moins une pièce.

31. Dispositif (100) selon l'une des revendications 1 à 30, **caractérisé en ce que** le dispositif (100) présente au moins un dispositif de mesure de niveau (17a, 17b) pour mesurer un niveau présent dans le réservoir d'agent désinfectant (4), pour mesurer un niveau présent dans le récipient de réception (13) et/ou pour mesurer un niveau présent dans le réservoir de produit de rinçage (15).

32. Dispositif (100) selon l'une des revendications précédentes, **caractérisé en ce que** le réservoir d'agent désinfectant (4), le réservoir de produit de rinçage (15) et/ou le multi-récipient (50) est réalisé sous forme de récipient jetable interchangeable ou est réalisé sous forme de récipient intégré de manière fixe dans le dispositif (100).

33. Dispositif (100) selon l'une quelconque des revendications précédentes, **caractérisé en ce que** le plateau à cloche (1),
- en fonctionnement, présente une vitesse de rotation d'au moins 30 000 tours par minute,
- présente une capacité de pulvérisation de plus de 50 millilitres par minute,
- présente un volume d'air circulé de plus de 0,3 mètre cube par minute, et/ou présente une arête d'éjection annulaire à disque à cloche (E) pour l'éjection de l'agent désinfectant et l'arête d'éjection à disque à cloche (E) présente de préférence un diamètre compris entre 40 mm et 120 mm.

34. Dispositif (100) selon l'une quelconque des revendications précédentes, **caractérisé en ce qu'**une surface intérieure (la) du plateau à cloche (1) présente
- un angle intérieur (alpha) compris entre 5° et 45°, et/ou
- présente une zone de bordure, la zone de bordure se raccordant à l'arête d'éjection du disque à cloche (E) et présentant une largeur (X) comprise entre 0,05 mm et 10 mm et seule la zone de bordure étant pourvue d'une structure de surface pour l'agent désinfectant.

35. Dispositif (100) selon l'une des revendications précédentes, **caractérisé en ce que** le dispositif (100) présente au moins un dispositif de mesure de l'espace pour mesurer l'espace, un processus de désinfection du dispositif (100) étant effectué en fonction des données de mesure du dispositif de mesure de l'espace ou un lancement d'un processus de désinfection du dispositif (100) étant empêché.

36. Dispositif (100) selon l'une quelconque des revendications précédentes, **caractérisé en ce que** le dispositif (100) présente une bague de guidage de gaz (R) comportant au moins un orifice de fuite (N) pour délivrer un gaz, et la bague de guidage de gaz (R) sert à guider le gaz sur une surface extérieure du plateau à cloche (1), le gaz rencontrant l'agent désinfectant délivré par le plateau à cloche (1) pour former l'agent désinfectant et/ou réduire la taille de ses gouttelettes.

37. Dispositif (100) selon la revendication 36, **caractérisé en ce que** la bague de guidage de gaz (R) présente une pluralité d'orifices de fuite (N) agencés de manière annulaire et/ou enveloppe une base (B) du plateau à cloche (1) et une partie d'une surface extérieure (1b) du plateau à cloche (1).

38. Dispositif (100) selon l'une des revendications précédentes, **caractérisé en ce que** le dispositif (100) comprend au moins un dispositif de détection pour détecter l'agent désinfectant, pour détecter le produit de rinçage et/ou pour détecter une vitesse de rotation du plateau à cloche (1).

39. Dispositif (100) selon l'une quelconque des revendications précédentes, **caractérisé en ce que** le dispositif (100) est adapté pour pulvériser l'agent désinfectant en gouttelettes dont la taille est comprise entre I pm et 50 pm, de préférence entre 3 pm et 10 pm.

40. Dispositif (100) selon l'une des revendications précédentes, **caractérisé en ce que** le dispositif (100) présente une unité de commande portable au moyen de laquelle le dispositif (100) peut être commandé par une liaison câblée ou sans fil.
